# EUROPEAN PATENT APPLICATION

(11) **EP 4 597 114 A1**
(43) Date of publication of application: **06.08.2025**
(21) Application number: 24305164.6
(22) Date of filing: 30.01.2024
(51) Int. Cl.: G01N 33/68

(54) **METHODS FOR IN VITRO PROGNOSIS, DIAGNOSIS, AND EVALUATION OF MUSCLE DYSFUNCTION AND/OR PROGNOSIS OF MUSCLE EFFICIENCY IN A SUBJECT**

(71) Applicant: Université Paris Cité, 75006 Paris (FR); Institut National de la Santé et de la Recherche Médicale, 75013 Paris (FR); Centre National de la Recherche Scientifique, 75016 Paris (FR)
(72) Inventor: HO, Tri Van Andrew, 75013 Paris (FR); FERREIRO, Ana, 75013 Paris (FR)
(74) Representative: Cabinet Beau de Loménie

(57) **Abstract**

The invention relates to an *in vitro* method for prognosticating, diagnosing and/or monitoring a muscle dysfunction in a subject and an *in vitro* method for evaluating and/or monitoring muscle efficiency in a subject, said method using specific ratios of tryptophan metabolites. The present invention also concerns a kit for implementing the *in vitro* methods according to the invention.

## Description

### FIELD OF THE INVENTION

The invention relates to an *in vitro* method for prognosticating and/or diagnosing muscle dysfunction in a subject and to an *in vitro* method for evaluating muscle efficiency in a subject. The present invention also concerns a kit for implementing the *in vitro* methods according to the invention.

### BACKGROUND OF THE INVENTION

Altered cell responses to stress such as oxidative stress or endoplasmic reticulum (ER) stress and cellular bioenergetics deficits are emerging as common alterations in a broad spectrum of muscle disorders. We can refer for instance to metabolic myopathies ([1]). It is well known that cellular metabolism and bioenergetic levels are directly correlated to muscle health and function.

SELENON-related myopathy is a muscle disease that causes muscle weakness, fatigue, loss of muscle mass and defective ATP production. SELENON-related myopathy can be used as a disease model of congenital myopathy since it is a monogenic disease due to mutations in SELENON, encoding a stress-defence protein; which allows to directly attribute any detectable changes in cellular and signalling pathways to this primary defect in cell response to stress, without confounding factors.

CREB and CEBP transcription factors play recognized roles in mediating a multitude of important functions for muscle physiology and regeneration ([2]; [3]; [4]; [5]). Impaired CREB activity in aged muscles, impacts mitochondrial functions and muscle strength ([6];[7]). CEBP (C/EBPo for CCAAT/enhancer-binding protein alpha) is a transcription factor involved in the regulation of various cellular processes, including cell differentiation, metabolism, and inflammation ([8]; [9]).

It has been demonstrated that SELENON mutations initially led to endoplasmic reticulum (ER) stress ([10]). Based on data interpretation, the Applicants made the first association of altered CREB and CEBP mediated transcriptional activities induced by the ER stress, to a congenital myopathy.

Using an unbiased transcriptomic analysis, the Applicants have further shown that disruption in CREB function accounts for more than a third of the gene expression changes in SELENON-related myopathy. Within this group of altered genes, the Applicants found through a bioinformatic pathway analysis, a high enrichment in the glycolytic pathway, lipid metabolism and more importantly tryptophan amino acid metabolism genes. The tryptophan metabolism sparked the Applicants' interest as it has never been described as implied in a congenital myopathy. More specifically, the Applicants demonstrated a connection between the endoplasmic reticulum (ER) stress and oxidative stress, which results in a down-regulation of KYNU (kynureninase) enzymatic activity in the tryptophan metabolism pathway in intrinsic muscle dysfunctions. These changes consequently lead to the accumulation of kynurenine (KYN) and kynurenic acid (KA), referred to as uremic biomarkers, upstream of KYNU catalytic activity. The Applicants have shown that the levels of KYN and KA are elevated in myoblasts derived from patients expressing SELENON mutations and suffering from a form of congenital myopathy, as well as in skeletal muscle tissues of the SELENON-deficient mouse model.

Thus, the Applicants have found that the loss of the enzyme kynureninase (KYNU) is critical to the breakdown of tryptophan (TRP) to produce NAD+ (oxidized nicotinamide adenine dinucleotide), an oxidized form of NAD (total NAD including oxidized (NAD+) and reduced (NADH) forms of nicotinamide adenine dinucleotides), and subsequently ATP (adenosine triphosphate), the currency of bioenergy. More particularly, the Applicants have found that in the case of deficiency of kynureninase (KYNU), kynurenine (KYN) metabolites accumulate in the muscles, where they can go through further breakdown to kynurenic acid (KA); affecting the downstream pathway via Quinolinic acid (QA) to produce NAD+ is blocked.

In other words, the Applicants have shown that while the level of quinolinic acid (QA) remains stable, elevated levels of kynurenine (KYN) and kynurenic acid (KA) are accompanied with a reduction of NAD+ (nicotinamide adenine dinucleotide)/ total NAD and/or ATP (adenosine triphosphate).

Furthermore, the Applicants have shown that these parameters are not only altered in congenital muscle dysfunctions but also in most muscle conditions characterized by a loss of muscle bioenergy, ultimately leading to muscle weakness and fatigue such as muscle aging, cachexia, and neuromuscular atrophy due to Spinal Muscular Atrophy (SMA).

In ageing muscles, kynurenine increase has been reported and proposed to have a negative impact on muscle function, but the mechanisms involved and the downstream consequences remain unclear ([10]). Moreover, in inherited neuromuscular diseases, the kynurenine pathway has never been disclosed or suggested as involved. KYN accumulation can lead to many biological consequences downstream. Based on the previously reported alterations of the kynurenine pathway in aging tissues and aged-related diseases, KYN accumulation would logically lead to an increase in quinolinic acid (QA) level ([11]; [12]). Indeed, it was shown that in ageing subjects with muscle dysfunction, the increased kynurenine pathway activity is associated with an increase of QA which can promote the production of reactive oxygen species (ROS) ([13]).

Consequently, elevated level of QA, likely due to multifactorial age-related changes, which would include a reduced Quinolinate phosphoribosyltransferase (QPRT) activity, has been associated with muscle dysfunction ([14]; [15]).

On contrary, the Applicants observed no significant change in QA or QPRT levels in the models of muscle dysfunction studied but showed a disruption of KYNU activity.

Interestingly, the ratio Tryptophan:Quinolinic acid (TRP/QA) is used as a baseline in the methods according to the invention as there is no deviation of this ratio between a reference subject and the subject suffering from muscle dysfunction and/or having weak muscle efficiency.

This finding is in opposition to the previous knowledge about muscle dysfunction as detailed above.

Therefore, the Applicants hereby propose i) a new *in vitro* method for prognosticating and/or diagnosing a muscle dysfunction in a subject and/or ii) an *in vitro* method for evaluating muscle efficiency in a subject, by determining several amount ratios of tryptophan pathway metabolites. According to a first aspect of the invention, the ratios of interest are i) Kynurenine:Tryptophan and/or Kynurenic acid:Tryptophan, and (ii) Quinolinic acid:Tryptophan. According to a second aspect of the invention, the ratios of interest are (i) Kynurenine:Quinolinic acid and/or Kynurenic acid:Quinolinic acid, and (ii) Quinolinic acid:Tryptophan.

### SUMMARY OF THE INVENTION

The inventors hereby propose a new approach i) for prognosticating and/or diagnosing and/or monitoring a muscle dysfunction, ii) for evaluating muscle efficiency, or iii) for monitoring muscle efficiency in a subject.

A first object of the invention concerns an *in vitro* method for prognosticating and/or diagnosing a muscle dysfunction in a subject, comprising:
a) measuring the amounts of kynurenine, tryptophan, kynurenic acid and quinolinic acid in a biological sample from the subject, and determining the ratios (i) Kynurenine:Tryptophan and/or Kynurenic acid:Tryptophan, and (ii) Quinolinic acid:Tryptophan;
b) comparing the ratios determined in step a) to a reference value;
c) attributing (i) an increase of the ratio Kynurenine:Tryptophan and/or Kynurenic acid:Tryptophan from the reference value, and (ii) no deviation of the ratio of Quinolinic acid:Tryptophan from the reference value to a muscle dysfunction in the subject.

A second object of the invention concerns an *in vitro* method for prognosticating and/or diagnosing a muscle dysfunction in a subject, comprising:
a) measuring the amounts of kynurenine, tryptophan, kynurenic acid and quinolinic acid in a biological sample from the subject, and determining the ratios (i) Kynurenine:Quinolinic acid and/or Kynurenic acid:Quinolinic acid, and (ii) Quinolinic acid:Tryptophan;
b) comparing the ratios determined in step a) to a reference value;
c) attributing (i) an increase of the ratio Kynurenine:Quinolinic acid and/or Kynurenic acid:Quinolinic acid from the reference value, and (ii) no deviation of the ratio of Quinolinic acid:Tryptophan from the reference value to a muscle dysfunction in the subject.

A third object of the invention concerns an *in vitro* method for evaluating the muscle efficiency in a subject, said method comprising:
a) measuring the amounts of kynurenine, tryptophan, kynurenic acid and quinolinic acid in a biological sample from the subject, and determining the ratios (i) Kynurenine:Tryptophan and/or Kynurenic acid:Tryptophan, and (ii) Quinolinic acid:Tryptophan;
b) comparing the ratios determined in step a) to a reference value;
c) attributing (i) an increase of the ratio Kynurenine:Tryptophan and/or Kynurenic acid:Tryptophan from the reference value, and (ii) no deviation of the ratio of Quinolinic acid:Tryptophan from the reference value to a weak muscle efficiency in the subject.

A fourth object of the invention concerns an *in vitro* method for evaluating the muscle efficiency in a subject, said method comprising:
a) measuring the amounts of kynurenine, tryptophan, kynurenic acid and quinolinic acid in a biological sample from the subject, and determining the ratios (i) Kynurenine:Quinolinic acid and/or Kynurenic acid:Quinolinic acid, and (ii) Quinolinic acid:Tryptophan;
b) comparing the ratios determined in step a) to a reference value;
c) attributing (i) an increase of the ratio Kynurenine:Quinolinic acid and/or Kynurenic acid:Quinolinic acid from the reference value, and (ii) no deviation of the ratio of Quinolinic acid:Tryptophan from the reference value to a weak muscle efficiency in the subject.

A fifth object of the invention concerns an *in vitro* method for monitoring a change in the diagnosis and/or prognosis of a muscle dysfunction in a subject, comprising:
a) implementing the method according to any of the first and second objects to the subject at one or more successive time points, whereby the diagnosis and/or prognosis of muscle dysfunction in the subject is determined at said successive time points; and
b) finding a presence or absence of a change between the diagnosis and/or prognosis of a muscle dysfunction in the subject at said successive time points as determined in step a).

A sixth object of the invention concerns an *in vitro* method for monitoring a change in the muscle efficiency in a subject, comprising:
a) implementing the method of any of the third and fourth objects to the subject at one or more successive time points, whereby the muscle efficiency in the subject is determined at said successive time points; and
b) finding a presence or absence of a change between the muscle efficiency in the subject at said successive time points as determined in step a).

A seventh object of the invention concerns a kit for prognosticating and/or diagnosing a muscle dysfunction and/or for evaluating muscle efficiency in a subject, comprising:
- one or more reagents for measuring the amounts of markers in a biological sample, wherein said markers are kynurenine, tryptophan, kynurenic acid and quinolinic acid,
- one or more containers suitable for mixing the biological sample with the one or more reagents,
- optionally, one or more surfaces which have an affinity for the markers which may be brought into contact with the biological sample,
- optionally, one or more suitable washing solutions,
- optionally, one or more components necessary for the detection of the markers, for example an enzyme and/or a substrate.

### DETAILED DESCRIPTION OF THE INVENTION

### Definitions

The terms "diagnosing" or "diagnosis" and "prognosticating" or "prognosis", as used herein, are used in the broadest sense, and are commonly used and are well-understood in medical and clinical practice. By means of further explanation and without limitation, "prognosticating" or "prognosis" refers to the determination of probability, risk or possibility of developing muscle dysfunction in a subject. By means of further explanation and without limitation, "diagnosing" or "diagnosis" refers to the determination of a probability or a possibility of having muscle dysfunction in a subject.

The term "patient" or "subject", as used herein, refers to a human or non-human mammal (such as a rodent (mouse, rat), a feline, a canine, or a primate) affected or likely to be affected with a muscle dysfunction and/or a loss of muscle efficiency. Preferably, the subject is a human, man or woman.

The term "muscle dysfunction", as used herein, refers to a pathological state that affects the human muscle system and its main manifestation is skeletal muscle weakness. In the context of the invention, the muscle dysfunction is associated with a bioenergetic deficiency of muscles which results from a lack of energy conversion from metabolic substrates by muscles. The terms "muscular dystrophy" and "neuromuscular disorders" fall under the umbrella of the term "muscle dysfunction". These dysfunctions correspond to a large group of conditions which affect either the skeletal muscles, such as but not limited to those in the body trunk, neck, diaphragm, facial muscles, muscles involved in mastication and swallowing, tongue, arms and legs, or the nerves which control the muscles.

For instance, the muscle dysfunction may result from:
i) a disease chosen from the group consisting of congenital myopathies, sarcopenia, cachexia, disuse atrophy, mitochondrial myopathies, metabolic myopathies, glycogen storage diseases, muscular dystrophies such as facioscapulohumeral muscular dystrophy (FSHD), myasthenia gravis, congenital myasthenic syndromes, inflammatory myopathies such as polymyositis, inclusion body myositis, myotonic dystrophy, cachexia, chronic fatigue syndrome, fibromyalgia, Guillain-Barré syndrome, spinal muscular atrophy, amyotrophic lateral sclerosis (ALS), peripheral neuropathy, drug-related myopathies, virus-related myopathies such as COVID-19 related, toxin-related flaccid paralysis such as Botulinum toxins related and/or
ii) a condition chosen from the group consisting of muscle with limited mobility, muscle trauma, muscle fatigue, muscle weakness, suboptimal muscle performance.

The term "muscle efficiency", as used herein, refers to a quantifiable measure of the capacity of a muscle or a group of muscles to perform mechanical work or generate force relative to the energy expended during contraction. This measure encompasses the ratio of useful work output or force generated by the muscle(s) to the energy input or metabolic expenditure, considering factors such as muscle fiber type, age of the subject, gender, demographic group etc. Muscle efficiency can be measured in various ways, depending on the specific context and the aspect of efficiency being evaluated. For example, by measuring the ratio of mechanical work output to the metabolic energy expended. It involves analyzing oxygen consumption and carbon dioxide production during muscle activity. This can be assessed through techniques like indirect calorimetry. Muscle efficiency can also be assessed by measuring exercise endurance (see below, under 'Muscle fatigue' section), force with dynamometry measurements, or by following the performance of everyday life activities (Real World Outcomes, RWO). The muscle efficiency may reflect the muscle strength and/or endurance and/or adaptation to training.

In the context of the invention, the term "muscle with limited mobility" refers to a condition with a reduced ability to perform the full range of normal body movements.

In the context of the invention, the term "weak muscle efficiency" refers to a condition with a low production of energy from metabolic substrates by muscle cells in comparison to a reference value, such as the energy produced by muscle cells in a healthy subject or a control subject. In the context of the invention, a weak muscle efficiency or muscle deficiency may result from muscle disease, muscle trauma, suboptimal muscle performance, reduced capacity to training adaptation, muscle with limited mobility and/or increased muscle fatigue.

The term "muscle fatigue", as used herein corresponds to an exercise-induced decrease in the ability of muscles to produce force, or to a decrease in maximal force or power production of muscles in response to contractile activity [16]. In physical examination, muscle fatigue may be defined by the time it takes for muscle weakness to manifest during repetitive or sustained muscle activities. Muscle fatigue may be measured with a dynamometer, which measures the maximum force exerted by a muscle or group of muscles, wherein a decrease in muscle strength compared to a reference value is taken as a sign of muscle fatigue. Muscle fatigue may also be measured with electromyography (EMG) which measures the electrical activity of muscles and can reveal muscle fatigue based on characteristic changes in the electrical signals of muscle fibers, wherein muscle fatigue is indicated by a sustained or repetitive muscle activity leading to an increase in the amplitude of the EMG signal and frequency variability.

In the context of the invention, the term "muscle trauma" refers to an injury to a muscle or a tendon which connects a muscle to a bone. Muscle trauma can correspond to minor injuries such as a muscle and/or a tendon overstretched or to more severe injuries such as partial or complete tears in these tissues, bruising of muscle tissue or lacerations caused by a direct blow or sharp object. Symptoms of muscle trauma may be i) pain or tenderness and/or ii) redness or bruising and/or iii) limited motion and/or iv) muscle spasms and/or v) swelling and/or vi) muscle weakness. Muscle trauma may be determined by physical examination and/or with electromyography (EMG) and/or by medical imaging techniques such as echography, magnetic resonance imaging and/or, computerized tomography (CT) scans..

A muscle weakness may be localized or global and corresponds to a diminished ability to generate muscle strength and/or tone and may be associated with a disruption in energy transfer, localized or global muscle loss. Muscle weakness may be determined by physical examination and/or with dynamometry, electromyography (EMG), and/or with bioelectrical impedance analysis. The severity of muscle weakness can be classified into different "grades" based on the Medical Research Council grading system for muscle strength, as follows [17], [18]:
Grade 0: No contraction or muscle movement,
Grade 1: Trace of contraction, but no movement at the joint,
Grade 2: Movement at the joint with gravity eliminated,
Grade 3: Movement against gravity, but not against added resistance,
Grade 4: Movement against external resistance with less strength than usual,
Grade 5: Normal strength.

Suboptimal muscle performance may correspond to lower muscle strength and/or endurance in comparison to a reference value which may corresponds to the value of muscle performance of a healthy subject or of a previous state in a subject. Suboptimal muscle performance may be measured by exercise and/or endurance tests.

The term "reference value", as used herein, means a value representing, depending on the object of the invention considered, A) (i) a diagnosis of no muscle dysfunction, (ii) a diagnosis of muscle dysfunction, (iii) a good prognosis of muscle dysfunction or (iv) a bad prognosis of muscle dysfunction; or B) (i) an evaluation of a normal muscle efficiency, (ii) an evaluation of weak muscle efficiency. A reference value may also encompass a reference range and/or ratio. More specifically, in the context of the invention the "reference value" may correspond to a value obtained in a healthy (i.e. not having a muscle dysfunction or a weak muscle efficiency) or a control subject, or to a value obtained at a certain previous time in a subject considered.

For the purposes of the invention, the term "marker" (or "biomarker") is an entity, such as tryptophan pathway metabolites, which can be used for i) the diagnosis and/or prognosis and/or monitoring a muscle dysfunction and/or ii) for evaluating and/or monitoring muscle efficiency in a subject. According to the invention, the biomarkers are chosen among Kynurenine (KYN), Tryptophan (TRP), Kynurenic acid (KA) Quinolinic acid (QA), Kynurenic acid oxidized nicotinamide adenine dinucleotide (NAD+), reduced nicotinamide adenine dinucleotide (NADH) and adenosine triphosphate (ATP).

The term "NAD+" refers to the oxidized form of nicotinamide adenine dinucleotides (NAD).

The term "total NAD" corresponds both to NAD+ and the reduced form of NAD (NADH).

The term "sample" or "biological sample", as used herein, includes any biological specimen obtained from a subject in which kynurenine, kynurenic acid, tryptophan, quinolinic acid, NAD+, NADH and ATP are in detectable quantities. The biological sample may be selected from the group consisting of blood, serum, plasma, urine, saliva and muscle sample. A muscle sample includes muscle biopsies.

The terms "amount", "quantity", and "level" are synonymous and generally well-understood in the art. The terms as used herein may particularly refer to an absolute quantification of a biomarker in a sample, or to a relative quantification of a biomarker in a sample (i.e. relative to another value such as relative to a reference value as taught herein), or to a range of values indicating a base-line expression of the biomarker. These values or ranges can be obtained from a single subject or from a group of subjects. An absolute quantity of a biomarker in a sample may be advantageously expressed as weight or as molar amount, or more commonly as a concentration. A relative quantity of a biomarker in a sample may be advantageously expressed as an increase or a decrease relative to (i.e. compared to) another value, such as relative to (i.e. compared to) a reference value.

The term "deviation" of a first value from a second value may generally encompass any direction, such as increase or decrease of a first value compared to a second value.

The term "treating" or "treatment" means reversing, alleviating, inhibiting the progression of, or preventing the disorder or condition to which such term applies, or one or more symptoms of such disorder or condition.

### Diagnosis and/or prognosis and/or monitoring muscle dysfunction

The present invention results from the advantages highlighted by the inventors that the measure of different ratios of metabolites of tryptophan's pathway makes it possible to diagnose and/or prognose a muscle dysfunction in a subject. The resulting applications are described below.

### Method 1

The invention relates to an *in vitro* method for prognosticating and/or diagnosing a muscle dysfunction in a subject, comprising:
a) measuring the amounts of kynurenine, tryptophan, kynurenic acid and quinolinic acid in a biological sample from the subject, and determining the ratios (i) Kynurenine:Tryptophan and/or Kynurenic acid:Tryptophan, and (ii) Quinolinic acid:Tryptophan;
b) comparing the ratios determined in step a) to a reference value;
c) attributing (i) an increase of the ratio Kynurenine:Tryptophan and/or Kynurenic acid:Tryptophan from the reference value, and (ii) no deviation of the ratio of Quinolinic acid:Tryptophan from the reference value to a muscle dysfunction in the subject.

The person skilled in the art will have no difficulty to measure the amounts of kynurenine, tryptophan, kynurenic acid and quinolinic acid by implementing methods known in the art.

The amounts of kynurenine, tryptophan, kynurenic acid and quinolinic acid can be measured with an immunoassay such as a competitive ELISA method. An example of ELISA is detailed in the example part, where 96-well plates are precoated with specific antigen to the solid phase of the microtiter plate. In this ELISA, the biologic sample is co-incubated with specific an antibody for 15-20h (overnight) at 2-8°C to allow time for competing with bound antigen in the ELISA plate for the limited binding sites on the antibody. After washing the unbound substance, the reaction may be detected by adding a secondary antibody conjugated with peroxidase enzyme and a chromogenic agent such as tetramethylbenzidine substrate, for the colorimetric readout at absorbance wavelength of 450nm. A reference wavelength between 620-650nm may be used as background reference control. The intensity of the colorimetric signal is inversely proportional to the amount of target antigen in the sample.

For example, the antibody used for implementing an immunoassay may be an antibody or fragment thereof that specifically binds to kynurenine, an antibody or fragment thereof that specifically binds to tryptophan, an antibody or fragment thereof that specifically binds to kynurenic acid, or an antibody or fragment thereof that specifically binds to quinolinic acid. Useful antibodies are available in ISE-220102 and ISE-2227 kits (provided by Immonusol, Bordeaux, France).

Other methods can also be implemented to measure the amount of kynurenine, tryptophan, kynurenic acid and quinolinic acid, such as Liquid Chromatography-Tandem Mass Spectrometry (LC-MS/MS) and High-Performance Liquid Chromatography (HPLC).

The specific ratios are calculated by dividing the amount of kynurenine, kynurenic acid and/or quinolinic acid with the amount of tryptophan measured in the subject.

These techniques of measuring the amounts of kynurenine, tryptophan, kynurenic acid and quinolinic acid can be implemented in all the methods of the present invention.

In some embodiments, the method of the invention also comprises the following measures:
a') in step a), also measuring the amounts of oxidized nicotinamide adenine dinucleotide (NAD+), total nicotinamide adenine dinucleotide (NAD) and adenosine triphosphate (ATP), and determining the ratio of NAD+: total NAD;
b') in step b), also comparing the ratio of NAD+: total NAD and the amount of ATP obtained in step a) to a reference value;
c') in step c), attributing (i) an increase of the ratio Kynurenine:Tryptophan and/or Kynurenic acid:Tryptophan from the reference value, (ii) no deviation of the ratio of Quinolinic acid:Tryptophan from the reference value, and (iii) a decrease of the ratio of NAD+: total NAD and/or of the amount of ATP from the reference value, to a muscle dysfunction in the subject.

The person skilled in the art will have no difficulty to measure the amounts of NAD+, NADH, NAD and ATP by implementing methods known in the art.

The amounts of ATP can be measured by submitting the cells to a direct lysis with a suitable detergent, followed by the interaction of the released ATP with a luciferin-luciferase and the measurement of the light emission at 562 nm. ATP level may be calculated on an ATP standard curve.

The amounts of NAD+ can be measured with an assay based on an enzymatic cycling reaction that reduces NAD+ to NADH, which then reacts with either a colorimetric or fluorometric probe. NAD+/NADH levels may be calculated based on a NAD+ standard curve.

The amount of NADH can be measured with an assay wherein in presence of NADH a reductase reduces the proluciferin reductase substrate to form luciferin. Luciferin is quantified using a recombinant bioluminescent luciferase, and the light signal produced is proportional to the amount of NADH in the sample.

The amount of total NAD corresponds to the amounts of NAD+ and NADH measured.

Other methods can also be implemented to measure the amount of NAD+, NADH and ATP, such as Cyclic Voltammetry which is based on the electrochemical technique where the redox state of NAD+ and NADH can be detected on the basis of their distinct electrochemical properties. Also, High-Performance Liquid Chromatography can be used to separate and quantify ATP via UV detection.

The specific ratios are calculated by dividing the amount of NAD+ with the amount of total NAD measured in the subject.

These techniques of measuring the amounts of kynurenine, tryptophan, kynurenic acid and quinolinic acid can be implemented in all the methods of the present invention.

In some embodiments, the ratio Kynurenine:Tryptophan and/or Kynurenic acid:Tryptophan in the sample from the subject is increased by at least 10%, preferably by at least 15%, preferably by at least 20%, more preferably by at least 25%, even more preferably by at least 30% compared to the reference value.

According to a preferred aspect, the ratio Kynurenine:Tryptophan and/or Kynurenic acid:Tryptophan in the sample from the subject is increased by at least 40%, preferably by at least 50%, more preferably by at least 60%, even more preferably by at least 75% compared to the reference value.

In some embodiments, the ratio of NAD+: total NAD and/or the amount of ATP in the sample from the subject is decreased by at least 10%, preferably by at least 15%, preferably by at least 20%, more preferably by at least 25%, even more preferably by at least 30% compared to the reference value.

According to a preferred aspect, the ratio of NAD+: total NAD and/or the amount of ATP in the sample from the subject is decreased by at least 40%, preferably by at least 50%, more preferably by at least 60%, even more preferably by at least 75% compared to the reference value.

### Method 2

Another object of the invention concerns an *in vitro* method for prognosticating and/or diagnosing a muscle dysfunction in a subject, comprising:
a) measuring the amounts of kynurenine, tryptophan, kynurenic acid and quinolinic acid in a biological sample from the subject, and determining the ratios (i) Kynurenine:Quinolinic acid and/or Kynurenic acid:Quinolinic acid, and (ii) Quinolinic acid:Tryptophan;
b) comparing the ratios determined in step a) to a reference value;
c) attributing (i) an increase of the ratio Kynurenine:Quinolinic acid and/or Kynurenic acid:Quinolinic acid from the reference value, and (ii) no deviation of the ratio of Quinolinic acid:Tryptophan from the reference value to a muscle dysfunction in the subject.

In some embodiments, this method also comprises the following measures:
a') in step a), also measuring the amounts of oxidized nicotinamide adenine dinucleotide (NAD+), total nicotinamide adenine dinucleotide (NAD) and adenosine triphosphate (ATP), and determining the ratio of NAD+: total NAD;
b') in step b), also comparing the ratio of NAD+: total NAD and the amount of ATP obtained in step a) to a reference value;
c') in step c), attributing (i) an increase of the ratios Kynurenine:Quinolinic acid and/or Kynurenic acid:Quinolinic acid from the reference value, (ii) no deviation of the ratio of Quinolinic acid:Tryptophan from the reference value, and (iii) a decrease of the ratio of NAD+: total NAD and/or of the amount of ATP from the reference value, to a muscle dysfunction in the subject.

The amounts of NAD+, NADH, NAD and ATP can be measured as detailed above.

In some embodiments, the ratio Kynurenine:Quinolinic acid and/or Kynurenic acid:Quinolinic acid in the sample from the subject is increased by at least 10%, preferably by at least 15%, preferably by at least 20%, more preferably by at least 25%, even more preferably by at least 30% compared to the reference value.

According to a preferred aspect the ratio Kynurenine:Quinolinic acid and/or Kynurenic acid:Quinolinic in the sample from the subject is increased by at least 40%, preferably by at least 50%, more preferably by at least 60%, even more preferably by at least 75% compared to the reference value.

In some embodiments, the ratio of NAD+: total NAD and/or the amount of ATP in the sample from the subject is decreased by at least 10%, preferably by at least 15%, preferably by at least 20%, more preferably by at least 25%, even more preferably by at least 30% compared to the reference value.

According to a preferred aspect, the ratio of NAD+: total NAD and/or the amount of ATP in the sample from the subject is decreased by at least 40%, preferably by at least 50%, more preferably by at least 60%, even more preferably by at least 75% compared to the reference value.

The methods 1 & 2 of the invention may be used in subjects who have not yet been diagnosed as having a muscle dysfunction (for example, in a preventative screening), or who have been diagnosed as having a muscle dysfunction, or who are suspected of having a muscle dysfunction (for example, display one or more symptoms characteristic of muscle dysfunction), or who are at risk of developing a muscle dysfunction. The methods may also be used to detect various stages of progression or severity of a muscle dysfunction.

In some embodiments of the methods 1 & 2 according to the invention, the muscle dysfunction results from:
i) a disease chosen from the group consisting of congenital myopathies, sarcopenia, cachexia, disuse atrophy, mitochondrial myopathies, metabolic myopathies, glycogen storage diseases, muscular dystrophies such as facioscapulohumeral muscular dystrophy (FSHD), myasthenia gravis, congenital myasthenic syndromes, inflammatory myopathies such as polymyositis, inclusion body myositis, myotonic dystrophy, cachexia, chronic fatigue syndrome, fibromyalgia, Guillain-Barré syndrome, spinal muscular atrophy, amyotrophic lateral sclerosis (ALS), peripheral neuropathy, drug-related myopathies, virus-related myopathies such as COVID-19 related, toxin-related flaccid paralysis such as Botulinum toxins related, and/or
ii) muscle with limited mobility, muscle trauma, muscle fatigue, muscle weakness, suboptimal muscle performance.

In a preferred embodiment of the methods 1 & 2 according to the invention, the muscle dysfunction results from a congenital myopathy chosen among SELENON-related myopathy, RYR1-related myopathy, and desminopathy.

In some embodiments of methods 1 & 2, the reference value represents a healthy subject.

In some embodiments, methods 1 & 2 further comprise a step comprising: subjecting the subject with a muscle dysfunction to a medical treatment, such as a therapeutic treatment or a prophylactic treatment or physical intervention. For instance, the prophylactic treatment or the physical intervention may be chosen among physical rehabilitation, exercise training, geriatric care or casting, bed rest or surgery or using assistive devices such as wheelchairs, crutches or other mobility aids.

### Method 3

According to another aspect the invention concerns an *in vitro* method for monitoring a change in the diagnosis and/or prognosis of a muscle dysfunction in a subject, comprising:
a) implementing the above method 1 or method 2, to the subject at one or more successive time points, whereby the diagnosis and/or prognosis of muscle dysfunction in the subject is determined at said successive time points; and
b) finding a presence or absence of a change between the diagnosis and/or prognosis of a muscle dysfunction in the subject at said successive time points as determined in step a).

In a preferred embodiment, the method further comprises an intermediate step b') comprising the comparison of the diagnosis and/or prognosis of muscle dysfunction in the subject at said successive time points as determined in step a).

In some embodiments, when method 1 is implemented for the monitoring, (i) an increase of the ratio Kynurenine:Tryptophan and/or Kynurenic acid:Tryptophan from a previous time point, and (ii) no deviation of the ratio of Quinolinic acid:Tryptophan from a previous time point, corresponds to a worsening of the muscle dysfunction in the subject.

According to a preferred aspect, the muscle dysfunction is worsened when the ratio Kynurenine:Tryptophan and/or Kynurenic acid:Tryptophan in the sample from the subject is increased by at least 10%, preferably by at least 15%, preferably by at least 20%, more preferably by at least 25%, even more preferably by at least 30% compared to the value from a previous time point.

According to a more preferred aspect, the muscle dysfunction is worsened when the ratio Kynurenine:Tryptophan and/or Kynurenic acid:Tryptophan in the sample from the subject is increased by at least 40%, preferably by at least 50%, more preferably by at least 60%, even more preferably by at least 75% compared to the value from a previous time point.

In some embodiments, when method 2 is implemented for the monitoring, (i) an increase of the ratio Kynurenine:Quinolinic acid and/or Kynurenic acid:Quinolinic acid from a previous time point, and (ii) no deviation of the ratio of Quinolinic acid:Tryptophan from a previous time point, corresponds to a worsening of the muscle dysfunction in the subject.

According to a preferred aspect, the muscle dysfunction is worsened when the ratio Kynurenine:Quinolinic acid and/or Kynurenic acid:Quinolinic acid in the sample from the subject is increased by at least 10%, preferably by at least 15%, preferably by at least 20%, more preferably by at least 25%, even more preferably by at least 30% compared to the value from a previous time point.

According to a more preferred aspect, the muscle dysfunction is worsened when the ratio Kynurenine:Quinolinic acid and/or Kynurenic acid:Quinolinic acid in the sample from the subject is increased by at least 40%, preferably by at least 50%, more preferably by at least 60%, even more preferably by at least 75% compared to the value from a previous time point.

In some embodiments, when method 1 or method 2 is implemented for the monitoring, a decrease of the ratio of NAD+: total NAD and/or of the amount of ATP from a previous time point corresponds to a worsening of the muscle dysfunction in the subject.

According to a more preferred aspect, the muscle dysfunction is worsened when the ratio of NAD+: total NAD and/or the amount of ATP in the sample from the subject is decreased by at least 10%, preferably by at least 15%, preferably by at least 20%, more preferably by at least 25%, even more preferably by at least 30% compared to the value from a previous time point.

According to a preferred aspect, the muscle dysfunction is worsened when the ratio of NAD+: total NAD and/or the amount of ATP in the sample from the subject is decreased by at least 40%, preferably by at least 50%, more preferably by at least 60%, even more preferably by at least 75% compared to the value from a previous time point.

In some embodiments, said change in the diagnosis and/or prognosis of muscle dysfunction in a subject, is monitored in the course of a medical treatment of said subject, such as a prophylactic treatment or a therapeutic treatment or physical intervention. For instance the prophylactic treatment or the physical intervention may be chosen among physical rehabilitation, exercise training, geriatric care or casting, bed rest or surgery or using assistive devices such as wheelchairs, crutches or other mobility aids.

In some embodiments of methods 1, 2 & 3, the biological sample is blood, urine, saliva or muscle sample. Indeed, the amounts of tryptophan metabolites remain stable in these samples making the methods according to the invention advantageously easy and quick to be performed.

### Evaluation and monitoring muscle efficiency

### Method 4

Another object of the invention concerns an *in vitro* method for evaluating the muscle efficiency in a subject, said method comprising:
a) measuring the amounts of kynurenine, tryptophan, kynurenic acid and quinolinic acid in a biological sample from the subject, and determining the ratios (i) Kynurenine:Tryptophan and/or Kynurenic acid:Tryptophan, and (ii) Quinolinic acid:Tryptophan;
b) comparing the ratios determined in step a) to a reference value;
c) attributing (i) an increase of the ratio Kynurenine:Tryptophan and/or Kynurenic acid:Tryptophan from the reference value, and (ii) no deviation of the ratio of Quinolinic acid:Tryptophan from the reference value to a weak muscle efficiency in the subject.

In some embodiments, this method also comprises the following measures:
a') in step a), also measuring the amounts of oxidized nicotinamide adenine dinucleotide (NAD+), total nicotinamide adenine dinucleotide (NAD) and adenosine triphosphate (ATP), and determining the ratio of NAD+: total NAD;
b') in step b), also comparing the ratio of NAD+: total NAD and the amount of ATP obtained in step a) to a reference value;
c') in step c), attributing (i) an increase of the ratio Kynurenine:Tryptophan and/or Kynurenic acid:Tryptophan from the reference value, (ii) no deviation of the ratio of Quinolinic acid:Tryptophan from the reference value, and (iii) a decrease of the ratio of NAD+: total NAD and/or of the amount of ATP from the reference value, to a weak muscle efficiency in the subject.

In some embodiments, the ratio Kynurenine:Tryptophan and/or Kynurenic acid:Tryptophan in the sample from the subject is increased by at least 10%, preferably by at least 15%, preferably by at least 20%, more preferably by at least 25%, even more preferably by at least 30% compared to the reference value.

According to a preferred aspect, the ratio Kynurenine:Tryptophan and/or Kynurenic acid:Tryptophan in the sample from the subject is increased by at least 40%, preferably by at least 50%, more preferably by at least 60%, even more preferably by at least 75% compared to the reference value.

In some embodiments, the ratio of NAD+: total NAD and/or the amount of ATP in the sample from the subject is decreased by at least 10%, preferably by at least 15%, preferably by at least 20%, more preferably by at least 25%, even more preferably by at least 30% compared to the reference value.

According to a preferred aspect, the ratio of NAD+: total NAD and/or the amount of ATP in the sample from the subject is decreased by at least 40%, preferably by at least 50%, more preferably by at least 60%, even more preferably by at least 75% compared to the reference value.

### Method 5

Another object of the invention concerns an *in vitro* method for evaluating the muscle efficiency in a subject, said method comprising:
a) measuring the amounts of kynurenine, tryptophan, kynurenic acid and quinolinic acid in a biological sample from the subject, and determining the ratios (i) Kynurenine:Quinolinic acid and/or Kynurenic acid:Quinolinic acid, and (ii) Quinolinic acid:Tryptophan;
b) comparing the ratios determined in step a) to a reference value;
c) attributing (i) an increase of the ratio Kynurenine:Quinolinic acid and/or Kynurenic acid:Quinolinic acid from the reference value, and (ii) no deviation of the ratio of Quinolinic acid:Tryptophan from the reference value to a weak muscle efficiency in the subject.

In some embodiments, this method also comprises the following measures:
a') in step a), also measuring the amounts of oxidized nicotinamide adenine dinucleotide (NAD+), total nicotinamide adenine dinucleotide (NAD) and adenosine triphosphate (ATP), and determining the ratio of NAD+: total NAD;
b') in step b), also comparing the ratio of NAD+: total NAD and the amount of ATP obtained in step a) to a reference value;
c') in step c), attributing (i) an increase of the ratios Kynurenine:Quinolinic acid and/or Kynurenic acid:Quinolinic acid from the reference value, (ii) no deviation of the ratio of Quinolinic acid:Tryptophan from the reference value, and (iii) a decrease of the ratio of NAD+: total NAD and/or of the amount of ATP from the reference value, to a weak muscle efficiency in the subject.

In some embodiments, the ratio Kynurenine:Quinolinic acid and/or Kynurenic acid:Quinolinic acid in the sample from the subject is increased by at least 10%, preferably by at least 15%, preferably by at least 20%, more preferably by at least 25%, even more preferably by at least 30% compared to the reference value, in a subject having a weak muscle efficiency in the subject.

According to a preferred aspect the ratio Kynurenine:Quinolinic acid and/or Kynurenic acid:Quinolinic in the sample from the subject is increased by at least 40%, preferably by at least 50%, more preferably by at least 60%, even more preferably by at least 75% compared to the reference value, in a subject having a weak muscle efficiency in the subject.

In some embodiments, the ratio of NAD+: total NAD and/or the amount of ATP in the sample from the subject is decreased by at least 10%, preferably by at least 15%, preferably by at least 20%, more preferably by at least 25%, even more preferably by at least 30% compared to the reference value, in a subject having a weak muscle efficiency in the subject.

According to a preferred aspect, the ratio of NAD+: total NAD and/or the amount of ATP in the sample from the subject is decreased by at least 40%, preferably by at least 50%, more preferably by at least 60%, even more preferably by at least 75% compared to the reference value in a subject having a weak muscle efficiency in the subject.

In some embodiments, the weak muscle efficiency results from a muscle disease, muscle trauma, muscle weakness, suboptimal muscle performance, muscle with limited mobility and/or muscle fatigue.

In some embodiments of these methods, the reference value represents a healthy subject meaning a subject with normal muscle efficiency.

As mentioned above, the term "muscle efficiency", as used herein, refers to a quantifiable measure of the capacity of a muscle or a group of muscles to perform mechanical work or generate force relative to the energy expended during contraction. This measure encompasses the ratio of useful work output or force generated by the muscle(s) to the energy input or metabolic expenditure, considering factors such as muscle fiber type, age of the subject, gender, etc.

Muscle efficiency can be measured in various ways, depending on the specific context and the aspect of efficiency being evaluated. For example, by measuring the ratio of mechanical work output to the metabolic energy expended. It involves analyzing oxygen consumption and carbon dioxide production during muscle activity. This can be assessed through techniques like indirect calorimetry. The muscle efficiency may reflect the muscle strength and/or endurance.

In the context of the invention a "weak muscle efficiency" refers to a condition with a low production of energy from metabolic substrates by muscle cells in comparison to a reference value, such as the energy produced by muscle cells in a healthy subject or a control subject.

In the context of the invention a "normal muscle efficiency" refers to a normal production of energy from metabolic substrates by muscle cells in comparison to a reference value, such as the energy produced by muscle cells in a healthy subject or a control subject.

For instance, a "normal muscle efficiency" may correspond to a maximal oxygen consumption (VO2) range (maximum amount of oxygen that the body can absorb and use during exercise) comprised between 30 and 50 mL/kg/min, preferably between 35 and 45 mL/kg/min, and more preferably between 30 and 40 mL/kg/min for a healthy adult.

For instance, a "normal muscle efficiency" may correspond to a respiratory exchange ratio (RER) (ratio between the metabolic production of carbon dioxide (CO2) and the uptake of oxygen (02)) values comprised between 0.7 (fat metabolism) to 1.0 (carbohydrate metabolism) during physical activity for a healthy adult.

### Method 6

Another object of the invention concerns an *in vitro* method for monitoring a change in the muscle efficiency in a subject, comprising:
a) implementing the methods 4 or 5 to the subject at one or more successive time points, whereby the muscle efficiency in the subject is determined at said successive time points; and
b) finding a presence or absence of a change between the muscle efficiency in the subject at said successive time points as determined in step a).

In a preferred embodiment, this method further comprises an intermediate step b'), between step a) and step b), comprising the comparison of the muscle efficiency in the subject at said successive time points as determined in step a).

In some embodiments, when method 4 is implemented for the monitoring, an increase of the ratio Kynurenine:Tryptophan and/or Kynurenic acid:Tryptophan from a previous time point, and (ii) no deviation of the ratio of Quinolinic acid:Tryptophan from a previous time point, corresponds to a worsening of the muscle efficiency in the subject.

According to a preferred aspect, the muscle efficiency is worsened when the ratio Kynurenine:Tryptophan and/or Kynurenic acid:Tryptophan in the sample from the subject is increased by at least 10%, preferably by at least 15%, preferably by at least 20%, more preferably by at least 25%, even more preferably by at least 30% compared to the value from a previous time point.

According to a more preferred aspect, the muscle efficiency is worsened when the ratio Kynurenine:Tryptophan and/or Kynurenic acid:Tryptophan in the sample from the subject is increased by at least 40%, preferably by at least 50%, more preferably by at least 60%, even more preferably by at least 75% compared to the value from a previous time point.

In some embodiments, when method 5 is implemented for the monitoring, an increase of the ratio Kynurenine:Quinolinic acid and/or Kynurenic acid:Quinolinic acid from a previous time point, and (ii) no deviation of the ratio of Quinolinic acid:Tryptophan from a previous time point, corresponds to a worsening of the muscle efficiency in the subject.

According to a preferred aspect, the muscle efficiency is worsened when the ratio Kynurenine:Quinolinic acid and/or Kynurenic acid:Quinolinic acid in the sample from the subject is increased by at least 10%, preferably by at least 15%, preferably by at least 20%, more preferably by at least 25%, even more preferably by at least 30% compared to the value from a previous time point.

According to a more preferred aspect, the muscle efficiency is worsened when the ratio Kynurenine:Quinolinic acid and/or Kynurenic acid:Quinolinic acid in the sample from the subject is increased by at least 40%, preferably by at least 50%, more preferably by at least 60%, even more preferably by at least 75% compared to the value from a previous time point.

In some embodiments, when method 1 or method 2 is implemented for the monitoring, a decrease of the ratio of NAD+: total NAD and/or of the amount of ATP from a previous time point, corresponds to a worsening of the muscle efficiency in the subject.

According to a more preferred aspect, the muscle efficiency is worsened when the ratio of NAD+: total NAD and/or the amount of ATP in the sample from the subject is decreased by at least 10%, preferably by at least 15%, preferably by at least 20%, more preferably by at least 25%, even more preferably by at least 30% compared to the value from a previous time point.

According to a preferred aspect, the muscle efficiency is worsened when the ratio of NAD+: total NAD and/or the amount of ATP in the sample from the subject is decreased by at least 40%, preferably by at least 50%, more preferably by at least 60%, even more preferably by at least 75% compared to the value from a previous time point.

In some embodiments, the change in the muscle efficiency of the subject is monitored in the course of a medical treatment of said subject, such as a prophylactic treatment or a therapeutic treatment or physical intervention. For instance, the prophylactic treatment or the physical intervention may be chosen among physical rehabilitation, exercise training, geriatric care or casting, bed rest or surgery or using assistive devices such as wheelchairs, crutches or other mobility aids.

According to an aspect, the methods 4, 5 & 6 are particularly interesting for monitoring a change of the muscle efficiency in a subject before, during and/or after a spatial travel or a sport training.

In some embodiments of the methods 4, 5 & 6, the biological sample is blood, urine, saliva or muscle sample. Indeed, the amounts of tryptophan metabolites remain stable in these samples making the methods according to the invention advantageously easy and quick to be performed.

### Kit

Another object of the invention concerns a kit for prognosticating and/or diagnosing a muscle dysfunction and/or for evaluating muscle efficiency in a subject, comprising:
- one or more reagents for measuring the amounts of markers in a biological sample, wherein said markers are kynurenine, tryptophan, kynurenic acid, quinolinic acid, , NAD+, NADH and/or ATP,
- one or more containers suitable for mixing the biological sample with the one or more reagents,
- optionally, one or more surfaces which have an affinity for the markers which may be brought into contact with the biological sample,
- optionally, one or more suitable washing solutions,
- optionally, one or more components necessary for the detection of the markers, for example an enzyme and/or a substrate.

In some embodiments, the one or more reagents are chosen among one or more antibodies and/or fragments thereof that specifically bind to one or more of the markers. For example, an antibody or fragment thereof that specifically binds to kynurenine, an antibody or fragment thereof that specifically binds to tryptophan, an antibody or fragment thereof that specifically binds to kynurenic acid, an antibody or fragment thereof that specifically binds to quinolinic acid, an antibody or fragment thereof that specifically binds to NAD+, an antibody or fragment thereof that specifically binds to NADH and/or an antibody or fragment thereof that specifically binds to ATP.

In some embodiments, the surface may be an ELISA template, or the surface may further incorporate or be adapted for use with advanced detection technologies such as Biacore technology based on surface plasmon resonance (SPR) and electrochemical detection methods. For instance, in ELISA, the surface may be a 96-well plate format, pre-coated with antigens corresponding to the markers of interest, said wells being configured to receive the biological sample and facilitate competitive binding between said antigens and said markers present in the biological sample with respect to the specific antibodies. The Biacore technology utilizes SPR to monitor the binding events occurring on the sensor surface in real-time, without the need for labels. Additionally, embodiments may integrate electrochemical detection methods, which rely on the measurement of electrical changes induced by the binding events on the surface.

In some embodiments, the washing solution is formulated to remove unbound substances from the reaction wells.

In some embodiments, the components necessary for the detection of the markers may be secondary antibodies conjugated with peroxidase enzyme, said secondary antibodies being configured to recognize and bind to the specific antibodies when the latter are attached to the immobilized antigens on the surfaces of the reaction wells.

In some embodiments, the kit according to the invention may comprise one or more reagents including a chromogenic substrate, specifically tetramethylbenzidine, formulated to produce a colorimetric signal at an absorbance wavelength of 450nm when acted upon by the peroxidase enzyme, wherein the intensity of said colorimetric signal is inversely proportional to the amount of target markers present in the biological sample.

In some embodiments, the Biacore technology involves immobilizing one of the interacting partners (e.g., an antibody) on a gold film sensor chip. The interaction with the binding partner (e.g., an antigen) causes a change in the refractive index near the surface, which is detected and quantified by SPR analysis.

In some embodiments, the surface for electrochemical detection may be modified with the graphite electrodes with high affinity for the binding of the biological molecules of interest, such as Kynurenin or Trypophan to the surface can result in measurable changes in current, voltage, or impedance. These changes are directly related to the concentration and interactions of the biological molecules of interest to provide a quantifiable means of detection.

### Computer-implemented methods

The methods according to the first, second, third, fourth, fifth and sixth objects may be implemented by a system. Such system may be realized in digital electronical circuity, integrated circuity, specially designed ASICs (application-specific integrated circuits), computer hardware, firmware, software, and/or combinations thereof. These implementations can include one or more computer programs that are executable and/or interpretable on a programmable system including at least one programmable processor, which may be special or general purpose, coupled to receive data and instructions from, and to transmit data and instructions to, a storage system, at least one input device, and at least one output device. Such computer programs may include machine instructions for a programmable processor, and may be implemented in any form of programming language. The computer programs may be in the form of source code, object code, or code intermediate between source code and object code, such as in partially complied form, or in any other desirable form. Therefore, the methods described herein may be computer-implemented methods. The computer-implemented methods may involve a computer program. Such computer program may comprise instructions for executing at least one step of the computer-implemented method according to the invention.

For example, the present description provides a computer-implemented method, in connection with the first object, for prognosticating and/or diagnosing a muscle dysfunction in a subject or, in connection with the third object, for evaluating the muscle efficiency in a subject, comprising:
a) measuring the amounts of kynurenine, tryptophan, kynurenic acid and quinolinic acid in a biological sample from the subject, and determining the ratios (i) Kynurenine:Tryptophan and/or Kynurenic acid:Tryptophan, and (ii) Quinolinic acid:Tryptophan;
b) comparing the ratios determined in step a) to a reference value;
c) attributing (i) an increase of the ratio Kynurenine:Tryptophan and/or Kynurenic acid:Tryptophan from the reference value, and (ii) no deviation of the ratio of Quinolinic acid:Tryptophan from the reference value to a muscle dysfunction in the subject in connection with the first object, or to a weak muscle efficiency in the subject in connection with the third object.

For example, the present description also provides a computer-implemented method, in connection with the first object, for prognosticating and/or diagnosing a muscle dysfunction in a subject or, in connection with the third object, for evaluating the muscle efficiency in a subject, comprising:
a) obtaining measures of the amounts of kynurenine, tryptophan, kynurenic acid and quinolinic acid in a biological sample from the subject, and determining the ratios (i) Kynurenine:Tryptophan and/or Kynurenic acid:Tryptophan, and (ii) Quinolinic acid:Tryptophan;
b) comparing the ratios determined in step a) to a reference value;
c) attributing (i) an increase of the ratio Kynurenine:Tryptophan and/or Kynurenic acid:Tryptophan from the reference value, and (ii) no deviation of the ratio of Quinolinic acid:Tryptophan from the reference value to a muscle dysfunction in the subject in connection with the first object, or to a weak muscle efficiency in the subject in connection with the third object.

For example, the present description also provides a computer-implemented method, in connection with the second object, for prognosticating and/or diagnosing a muscle dysfunction in a subject or, in connection with the fourth object, for evaluating the muscle efficiency in a subject, comprising:
a) measuring the amounts of kynurenine, tryptophan, kynurenic acid and quinolinic acid in a biological sample from the subject, and determining the ratios (i) Kynurenine:Quinolinic acid and/or Kynurenic acid:Quinolinic acid, and (ii) Quinolinic acid:Tryptophan;
b) comparing the ratios determined in step a) to a reference value;
c) attributing (i) an increase of the ratio Kynurenine:Quinolinic acid and/or Kynurenic acid:Quinolinic acid from the reference value, and (ii) no deviation of the ratio of Quinolinic acid:Tryptophan from the reference value to a muscle dysfunction in the subject in connection with the second object, or to a weak muscle efficiency in the subject in connection with the fourth object.

For example, the present description also provides a computer-implemented method, in connection with the second object, for prognosticating and/or diagnosing a muscle dysfunction in a subject or, in connection with the fourth object, for evaluating the muscle efficiency in a subject, comprising:
a) obtaining measures of the amounts of kynurenine, tryptophan, kynurenic acid and quinolinic acid in a biological sample from the subject, and determining the ratios (i) Kynurenine:Quinolinic acid and/or Kynurenic acid:Quinolinic acid, and (ii) Quinolinic acid:Tryptophan;
b) comparing the ratios determined in step a) to a reference value;
c) attributing (i) an increase of the ratio Kynurenine:Quinolinic acid and/or Kynurenic acid:Quinolinic acid from the reference value, and (ii) no deviation of the ratio of Quinolinic acid:Tryptophan from the reference value to a muscle dysfunction in the subject in connection with the second object, or to a weak muscle efficiency in the subject in connection with the fourth object.

For example, the present description also provides an in vitro computer-implemented method for monitoring a change in the diagnosis and/or prognosis of a muscle dysfunction in a subject, comprising:
a) implementing the method as described above to the subject at one or more successive time points, whereby the diagnosis and/or prognosis of muscle dysfunction in the subject is determined at said successive time points; and
b) finding a presence or absence of a change between the diagnosis and/or prognosis of a muscle dysfunction in the subject at said successive time points as determined in step a).

For example, the present description also provides an in vitro computer-implemented method for monitoring a change in the muscle efficiency in a subject, comprising:
a) implementing the method as described above to the subject at one or more successive time points, whereby the muscle efficiency in the subject is determined at said successive time points; and
b) finding a presence or absence of a change between the muscle efficiency in the subject at said successive time points as determined in step a).

The present description also relates to a computer program comprising instructions for carrying out the steps of the computer-implemented methods disclosed herein. Said program may be executed by a computer.

The present description also relates to a computer-readable recording medium on which is recorded a computer program comprising instructions for carrying out the steps of the computer-implemented methods disclosed herein. The computer-readable recording medium referred to in this disclosure may be any entity or device capable of storing the program. For example, the computer-readable recording medium may comprise a storage medium, such as a ROM, for example a CD ROM or a microelectronic circuit ROM, or a magnetic recording medium, for example a floppy disk or a hard disk. The computer-readable recording medium may correspond to a transmissible medium such as an electrical or optical signal, which may be conveyed via an electrical or optical cable, by radio or by other means. In particular, the program can be downloaded from an Internet-type network.

The following examples illustrate the present invention without limiting the scope of the invention to said examples.

### FIGURES

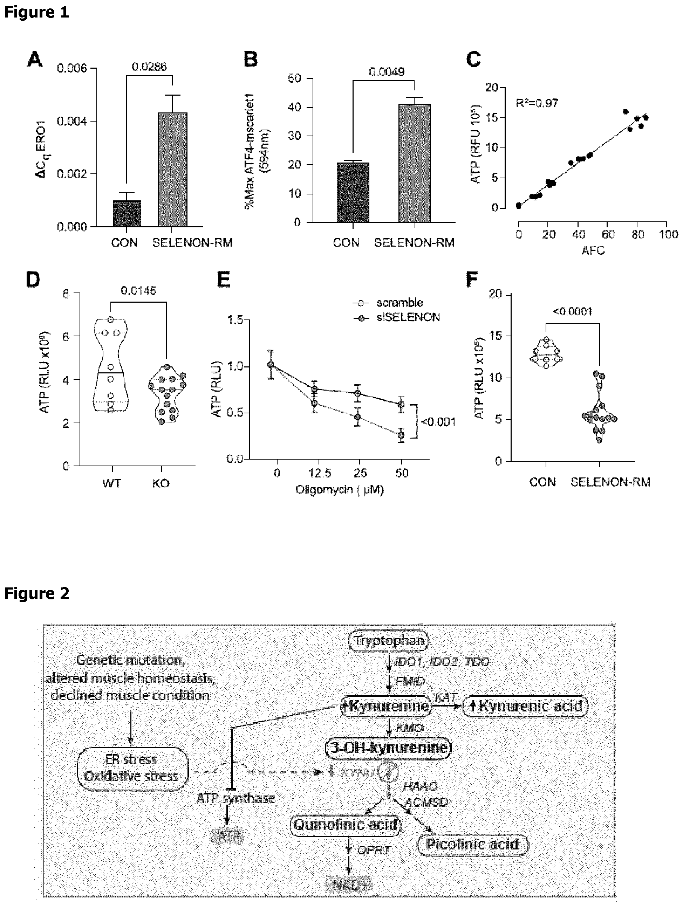

**Figure 1** is relative to ER stress and ATP reduction detected in SELENON-RM myoblast samples. Increase in ER stress markers in SELENON-RM myoblast samples is observed. Increase in ERO1 expression is shown by qPCR (Figure 1 A) and ATF4 signal (Figure 1 B) is revealed by the fluorescent biosensor encoded by the transfected pLHCX-ATF4 mScarlet NLS construct (a gift from David Andrews, Addgene plasmid # 115970 SEQ ID N°3) in the primary myoblast cultures. Figure 1 C represents the correlation between ATP levels and cell number determined by AFC fluorescence reporter (7-amino-4-trifluoromethylcoumarin, Abcam). The scatter plot represents the ATP level (Y-axis) in relation to the cell number determined by the fluorescence of the AFC (X-axis). Measurement of ATP levels in selenon-/- (KO) compared to control (WT) group (Figure 1 D), in siRNA Knockdown of SELENON (siSELENON) in control human myoblast cultures under increasing Oligomycin treatment in comparing to non-target scramble (scramble) condition (Figure 1 E) and in myoblasts derived from SELENON-RM and healthy control biopsies (Figure 1 F). Statistical significance in A, B, D and F was determined by t-test analysis and in E by 2-way ANOVA test with Dunnett post-hoc multiple comparison test correction.
**Figure 2** is a schematic representation of the kynurenine pathway. Tryptophan is initially converted into kynurenine (KYN) by a series of enzymatic reaction. Subsequently, kynurenine can be converted into kynurenic acid (KA), quinolinic acid (QA), or picolinic acid by the enzyme kynureninase (KYNU).

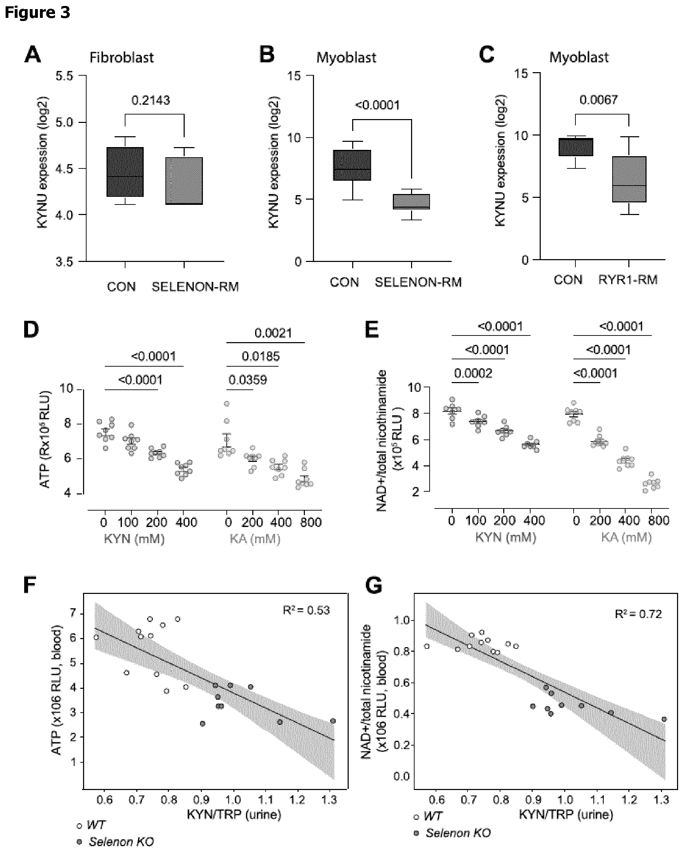

**Figure 3** represents the association of ER stress in SELENON-RM with bioenergetic deficiency and disruption of Kynurenine metabolism specific to myoblasts. KYNU expression is measured in human SELENON-RM fibrobast (Figure 3 A), myoblast (Figure 3 B) and RYR1-RM myoblast (Figure 3 C). Measurement of ATP (D) and NAD+/total nicotinamide (E) in healthy myoblast cultures with increasing kynurenine (KYN) or kynurenic acid (KA) supplement for 24h. Mann-Whitney (2-tailed) test was performed for (Figure 3 A-C) and one-way ANOVA test was performed with Holm-Šídák post-hoc correction for D, E. Inversed correlation of ATP level (Figure 3 F) and NAD+/total nicotinamide (Figure 3 G) measured in blood to the KYN/TRP ratio measured in urine samples, obtained from *Selenon-*/*-* (KO) mouse and wildtype (WT) littermates.

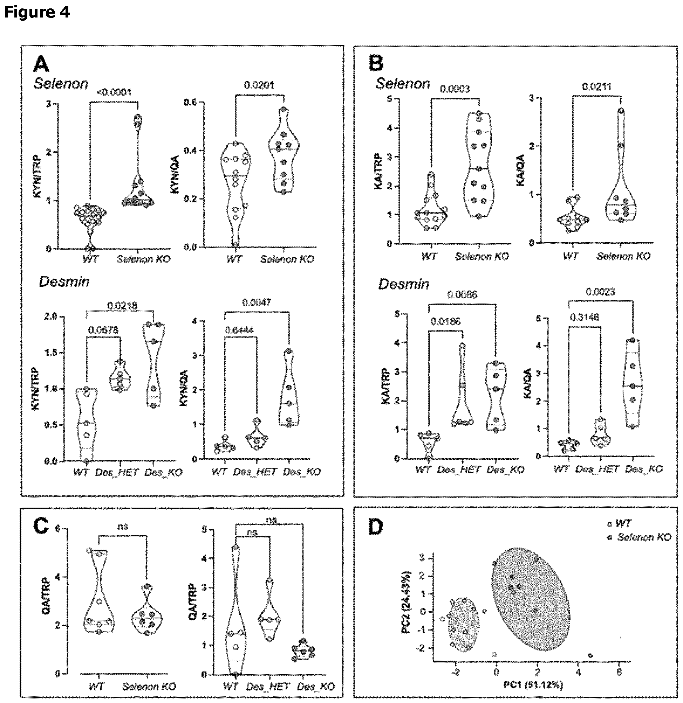

**Figure 4** represents the evaluation of kynurenine biomarkers in diseases associated with weak muscle conditions. Figure 4 A represents a comparative analysis of KYN to TRP or QA Ratios in urine samples of *Selenon-*/*-* and *Desmin-*/*-* Mouse Models. Figure 4 B represents a comparative Analysis of KA to TRP or QA ratios in urine samples of *Selenon-*/*-* (*Selenon KO*)*, Desmin+*/*-(Des_Het*) and *Desmin-*/*-* (*Des_KO*) mouse models. Figure 4 C represents QA/Trp Ratio evaluating consistencies across knockout models and controls. Figure 4 D represents a segregation of WT from *Selenon-*/*-* groups by PCA analysis of different combination of ratio obtained from KYN, KA, TRP and QA measured in urine samples. Unpaired t-test was performed for 2 sample comparison and Kruskal-Wallis test with Dunn's post hoc correction was performed for multiple sample comparison. P-value is indicated for each pair-wise comparison.

### EXAMPLES

### Example 1: Evaluation of endoplasmic reticulum stress (ER) level and bioenergetic deficits in SELENON-RM myoblast samples

The increase in ER stress markers in SELENON-related myopathy (SELENON-RM) myoblast samples was studied. Endoplasmic reticulum oxidoreductase 1(ERO1) and the activating transcriptor factor 4 (ATF4) were used as proxy markers to ER stress. To investigate a potential link between ER stress and defective bioenergy production in this disease model, a high-throughput method has been devised to measure ATP level in a 96-well plate based on luminescent signal intensity as function of ATP level.

More specifically, the increase in ERO1 expression was measured by qPCR (see Figure 1A) and ATF4 signal was revealed by the fluorescent biosensor encoded by the transfected pLHCX-ATF4 mscarlet NLS construct (a gift from David Andrews, Addgene plasmid # 115970 SEQ ID N°3) in the primary myoblast cultures (see Figure 1B), ATP levels were evaluated via luciferase activity (Figure 1C).

### Materials and Methods

### a) Human primary cultures

The myoblasts were derived from muscle biopsies obtained from patients diagnosed with SELENON mutation by genomic DNA sequencing (SELENON-RM) and healthy donors (control), after informed consent for biomedical research, by Genethon Biobank and Cochin Biobank in France. Primary myoblasts were further enriched for CD56+ surface marker via FACS and were maintained in collagen coated plates and myogenic cell culture medium containing DMEM/F10 (50:50), 20% FBS, 2.5 ng·mL-1 fibroblast growth factor-2 (FGF-2 also known as bFGF) and 1% penicillin-streptomycin

### b) Measurement of ERO1 expression by qPCR

Total RNA was isolated using the RNeasy Micro Kit (Qiagen). cDNA from total RNA from each sample was reverse-transcribed using the SensiFAST cDNA Synthesis Kit (Bioline). RT-PCR was performed using SYBR Green PCR Master Mix (Applied Biosystems) in an ABI 7900HT Real-Time PCR System (Applied Biosystems). Samples were amplified at 95 °C for 10 min and then 40 cycles at 95 °C for 15 s and 58 °C for 1 min. The delta CT was determined by calculating 2^{^(-deltaCT),} where deltaCT is the difference of target to 18S housekeeping qPCR value. The following ERO1 primer sequences were used:
SEQ ID N°1: hs-ERO1 Forward GGC TTC TGG TCA AGG GAC AA
SEQ ID N°2: hs-ERO1 Reverse TGC TTG CAT GTA GGC CAG ATA

### c) Measurement of ATF4 ER stress

The ER stress was measured via an ATF4 fluorescent readout marker. AFC is non-fluorescent until cleaved by metabolic active cell to emit a green fluorescence.

An expression plasmid pLHCX-ATF4 mScarlet NLS, encoding the fusion protein ATF4-mscarlet (a gift from Dr. David Andrews; Addgene#115970 SEQ ID N°3), was transfected using lipofectamine 3000 reagent (Thermofisher) following the standard protocol. At the 3d day post-transfection, 1×10⁴ cells were seeded to 96-well plate. ATF4 response was detected 24h after seeding by measuring the mscarlet fluorescent signal at 570-530nm wavelength using 561nm excitation wavelength via the Flexstation 3 (Molecular devices). The signal was normalized by the total maximum signal from the transfected cells obtained after incubating with hydrogen peroxide (H2O2, 100µM) for 16h. n=4 technical replicates for each condition were used to measure the signal.

### d) ATP measurement

ATP levels were evaluated via luciferase activity, in which the introduced luciferase enzyme utilizes cellular ATP and luciferin to produce detectable light, thereby providing a measure of ATP concentration.

1-4×10³ cells were seeded in each 96-well plate and cultured in myoblast media for 16h before performing the assay. The cultures were subsequently stimulated with Oligomycin (0-50uM) for 4h. Viable cells were determined by incubating the cells for 2h at 37°C with a fluorogenic cell-permeant peptide Gly-Phe-7-Amino-4-Trifluoromethylcoumarin peptide (MPBio) at 390nm ex /505nm. The ATP level (in relative luminescent unit RLU) in each culture was measured using the luciferase kit (Promega) with the Centro microplate luminometer (Berthold) following the manufacturer's protocol. n=4 technical replicates were performed for each condition with at least 3 independent experiments.

### e) SiRNA knockdown

Using the Silencer^{®} siRNA Transfection II Kit, SELENON was targeted for knockdown in human primary myoblast cultures. Once the cells achieved 50-70% confluency, they were transfected with siRNAs specific to human SELENON expression at a final concentration of 100 nM. The siRNAs were mixed with the Silencer^{®} siRNA Transfection II reagent, incubated to allow complex formation, and then added to the cells. Following a 24-72 hour incubation period, the efficiency of the knockdown was assessed using qPCR. The downstream experiments were proceeded upon confirming expression to be less 50% compared to untreated control.

### f) Statistical analysis

Statistical significance in A, B, D and F was determined by t-test analysis and in E by 2-way ANOVA test with Dunnett post-hoc multiple comparison test correction.

### Results

Using ERO1 and ATF4 as proxy markers to ER stress, it has been shown that SELENON-RM exhibited a significant higher level compared to the control (Fig.1 A, B). ER stress has been established as a factor that influences mitochondrial bioenergetics. The presented data corroborate this, suggesting that SELENON-RM exhibits a deficiency in bioenergetic production, which appears to be a consequence of ER stress.

To investigate a potential link between ER stress and defective bioenergy production in this disease model, a high-throughput technique has been developed to measure ATP level in a 96-well plate based on luminescent signal intensity as function of ATP level. These data allows observing that the ATP measurement based on luminescent readout was within the linear and detectable range and was directly proportional to the cell number seeded in the microwells as measured by AFC (Fig. 1C). It has been observed that ATP was significantly decreased in SELENON-deficient mouse model (KO, Fig. 1D) and in primary myoblast cultures, either knocked-down for the SELENON gene (Fig. 1E) or derived from SELENON-RM patients (Fig. 1F), compared to control cultures.

### Example 2: Evaluation of ER stress in SELENON-RM and its association with bioenergetic deficiency and disruption of kynurenine metabolism specific to myoblasts.

### Materials and Methods

### a) ATP and NAD+ measurements

1-4×10³ cells were seeded in each 96-well plate and cultured in myoblast media for 16h before performing the assay. The cultures were subsequently stimulated with Oligomycin (0-50uM) for 4h. Viable cells were determined by incubating the cells for 2h at 37°C with a fluorogenic cell-permeant peptide Gly-Phe-7-Amino-4-Trifluoromethylcoumarin (C₂₁H₁₈F₃N₃O₄) peptide (MPBio) at 505nm using 390nm excitation wavelength. The ATP and NAD levels (in relative luminescent unit RLU) in each culture were measured using the luciferase kit (Promega) with the Centro microplate luminometer (Berthold) following the manufacturer's protocol. n=4 technical replicates were performed for each condition with at least 3 independent experiments.

### b) Human primary cultures

The myoblasts were derived from diagnostic muscle biopsies while fibroblasts were isolated from skin biopsies obtained from patients diagnosed with SELENON mutation (SELENON-RM) or RYR1 mutation (RYR1-RM) by genomic DNA sequencing and healthy donors (control), after informed consent for biomedical research, by Genethon Biobank and Cochin Biobank in France.

Primary myoblasts were further enriched for CD56+ surface marker via FACS and were maintained in collagen coated plates and myogenic cell culture medium containing DMEM/F10 (50:50), 20% FBS, 2.5 ng·mL⁻¹ fibroblast growth factor-2 (FGF-2 also known as bFGF) and 1% penicillin-streptomycin.

Fibroblasts were cultured in Dulbecco's Modified Eagle's Medium (DMEM) supplemented with 10% fetal bovine serum (FBS), 1% penicillin-streptomycin, and 1% L-glutamine. The cultures were maintained in a humidified incubator at 37°C with 5% CO₂.

### c) Sample collection

The urine samples (100ul volume per mouse) were collected by immobilizing the mouse by holding onto its back to the top of the metal grid lit of the mouse cage. The urine from the spontaneous urination was then collected from the clean weight boat placed below the lit. The voided urine was immediately aspirated with a pipette tip and store at -20°C until ready for ELISA.

The blood samples were collected via retroorbital route by gently inserting a sterile hematocrit capillary tube and allowed the blood to be directly drained into the Eppendorf tube; the blood samples were allowed to clot by leaving it undisturbed at room temperature within 15-30 minutes. The clot was then removed by centrifuging at 1,000-2,000 x g for 10 minutes in a refrigerated centrifuge. The supernatant (the serum fraction) was then stored in -80°C for the measurement of ATP and NAD+/total nicotinamide.

### d) Measurement of Tryptophan and its metabolites

The tryptophan (Trp), Kynurenin (KYN), Kynurenic acid (KA) and Quinolinic acid (QA) were measured by the Enzyme-Linked Immunosorbent Assay (ELISA) method, following the protocols that were provided by the ELISA kits purchased from ImmuSmol SAS (Bordeaux, France). Briefly, the assay was based on the competitive ELISA method where 96-well plates provided by the kit manufacturers were precoated with specific antigen to the solid phase of the microtiter plate. The urine samples after thawing to room temperature and diluting 5-fold with PBS were derivatized to increase stability and reactivity. The 25ul of derivatized samples was co-incubated with specific antibody for 15-20h (overnight) at 2-8°C to allow time for competing with bound antigen in the ELISA plate for the limited binding sites on the antibody. After washing the unbound substance, the reaction was detected by adding a secondary antibody conjugated with peroxidase enzyme and the chromogenic tetramethylbenzidine substrate for the colorimetric readout at absorbance wavelength of 450nm via the Flexstation 3 detector (Molecular devices). A reference wavelength between 620-650nm was obtained as background reference control. The intensity of the colorimetric signal was inversely proportional to the amount of target antigen in the sample.

For measurements across different experiments, the values of each independent experiments were normalized using min-max method and outliers were determined by Grubbs' method with alpha=0.05. Biased effect by age or gender was determined to be negligible based on the values and average calculations. The specific ratios were determined by dividing the normalized KYN or KA to TRP or QA. The ratio of QA to TRP was also determined and compared to that of WT ratio (reference value).

### e) Statistical analyses

Mann-Whitney (2-tailed) test was performed for (A-C) and one-way ANOVA test was performed with Holm-Šídák post-hoc correction for D, E.

### Results

No significant difference was detected in the expression of kynureninase (KYNU) in Selenon-RM fibroblasts (Fig. 3A). In contrast, a significant reduction was detected in Selenon-RM myoblasts (Fig. 3B), emphasizing the tissue-specific nature of this enzyme.

To determine if the observed KYNU expression changes extend to other muscle weakness-related disorders, the RYR1 (Ryanodine Receptor 1) disease model was analyzed. RYR1 mutations are known to result in an array of muscle disorders, with muscle weakness being a hallmark. Dysregulation in calcium homeostasis due to the RYR1 mutations is believed to contribute to this phenotype. Confirming our hypothesis, a significant reduction in KYNU expression in RYR1 patient-derived myoblasts was detected (Fig. 3C), suggesting that reduced KYNU expression may be a common molecular signature in muscle dysfunction conditions.

To establish causality between kynurenine (KYN) or kynurenic acid (KA) accumulation and the observed decline in cellular energy, healthy myoblast cultures were treated with increasing concentrations of KYN and KA. After a 24-hour incubation, a marked reduction in ATP levels (Fig. 3D) and NAD+ level were detected from total nicotinamide measurements (Fig. 3E). Furthermore, in analyses of *selenon* knockout (*selenon KO*) and *wild-type* (*WT*) control mice, an inverse correlation was observed between ATP levels (Fig. 3F) and NAD+ levels (Fig. 3G) in blood, and the kynurenine levels (normalized to tryptophan) measured in urine. This *in vivo* data mirrors *in vitro* observations, illustrating the detrimental effects of kyn and ka accumulation on cellular energy production.

### Example 3: Evaluation of kynurenine biomarkers in diseases associated with a weak muscle efficiency

In this study, a strategic combination of measurements was set up to analyze the kyurenin pathway and meet 2 objectives: (i) to discern if KYNU serves as a unique blockage point or if there is a generalized disruption throughout the pathway, and (ii) to pinpoint specific molecular markers that might indicate weak muscle efficiency. The ratios of KYN to TRP or QA (Fig. 4A) and KA to TRP or QA (Fig. 4B) in the *Selenon-*/*-* and *Desmin-*/*-* mouse models, were measured. To do so, a comparative analysis of KYN to TRP or QA ratios in urine samples *Selenon*-/- and *Desmin-*/*-* Mouse Models and a comparative analysis of KA to TRP or QA ratios in urine samples of *Selenon-*/*-* (*Selenon KO*)*, Desmin+*/*-*(*Des_Het*) and *Desmin-*/*-* (Des_KO) mouse models were made. In addition, a segregation of WT from *Selenon-*/*-* groups was made by PCA analysis of different combination of ratio obtained from KYN, KA, TRP and QA measured in urine samples.

### Materials and Methods

### a) Sample collection

The urine samples (100ul volume per mouse) were collected by immobilizing the mouse by holding onto its back to the top of the metal grid lit of the mouse cage. The urine samples from the spontaneous urination were then collected from the clean weight boat placed below the lit. The voided urine was immediately aspirated with a pipette tip and store at -20°C until ready for ELISA.

### b) Measurement of Tryptophan and its metabolites

The tryptophan (TRP), Kynurenin (KYN), Kynurenic acid (KA) and Quinolinic acid (QA) were measured by the Enzyme-Linked Immunosorbent Assay (ELISA) method, following the protocols that were provided by the ELISA kits purchased from ImmuSmol SAS (Bordeaux, France). Briefly, the assay was based on the competitive ELISA method where 96-well plates provided by the kit manufacturers were precoated with specific antigen to the solid phase of the microtiter plate. The urine samples after thawing to room temperature and diluting 5-fold with PBS were derivatized to increase stability and reactivity. The 25ul of derivatized samples was co-incubated with specific antibody for 15-20h(overnight) at 2-8°C to allow time for competing with bound antigen in the ELISA plate for the limited binding sites on the antibody. After washing the unbound substance, the reaction was detected by adding a secondary antibody conjugated with peroxidase enzyme and the chromogenic tetramethylbenzidine substrate for the colorimetric readout at absorbance wavelength of 450nm via the Flexstation 3 detector (Molecular devices). A reference wavelength between 620-650nm was obtained as background reference control. The intensity of the colorimetric signal was inversely proportional to the amount of target antigen in the sample.

For measurements across different experiments, the values of each independent experiments were normalized using min-max method and outliers were determined by Grubbs' method with alpha=0.05. Biased effect by age or gender was determined to be negligible based on the values and average calculations. The specific ratios were determined by dividing the normalized KYN or KA to TRP or QA. The ratio of QA to TRP was also determined and compared to that of WT ratio (reference value).

### c) Statistical analyses

Unpaired t-test was performed for 2 sample comparison and Kruskal-Wallis test with Dunn's post hoc correction was performed for multiple sample comparison. P-value is indicated for each pair-wise comparison.

### Results

The results obtained, robustly indicate that the primary blockage in bioenergy production in these muscle conditions (illustrating with *Selenon*-/- and *Desmin-*/*-* mouse models) occurs at the KYNU catalytic activity level. Supporting this assertion, it was found that the QA/TRP ratio in both knockout models remained largely unaltered from that of the control (Fig. 4C). Therefore, these data imply that deviations in KYN and KA are not simply a result of alterations in the TDO or IDO enzymes (which are responsible for the initial conversion of tryptophan into N-formylkynurenine, a precursor of KYN) nor from processes downstream of KYNU. Through Principal Component Analysis (PCA) analysis of urine sample ratios derived from KYN, KA, TRP, and QA measurements, distinction was effectively made between the WT and *Selenon-*/*-* groups, underlighting the potential of these molecular signatures as diagnostic indicators for the Selenon deficiency (Fig. 4D).

The introduction of the desmin knockout model, which models human desminopathy (a muscle condition originating from mutations in the DES gene) adds an insightful dimension to the study. The distinct muscle inadequate efficiency observed in this model enriches the present findings. Given the consistency of the findings across models with diverse etiologies, it appears that disruptions linked to KYNU can be used as specific biomarkers for muscle inadequate efficiency associated to various health conditions.

### BIBLIOGRAPHIC REFERENCES

[1] Johnson, T.A., Jinnah, H.A., and Kamatani, N. (2019). Shortage of Cellular ATP as a Cause of Diseases and Strategies to Enhance ATP. Frontiers in Pharmacology 10.
[2] Le Grand, F., Jones, A.E., Seale, V., Scimè, A., and Rudnicki, M.A. (2009). Wnt7a activates the planar cell polarity pathway to drive the symmetric expansion of satellite stem cells. Cell Stem Cell 4, 535-547. 10.1016/j.stem.2009.03.013.
[3]Ruffell, D., Mourkioti, F., Gambardella, A., Kirstetter, P., Lopez, R.G., Rosenthal, N., and Nerlov, C. (2009). A CREB-C/EBPbeta cascade induces M2 macrophage-specific gene expression and promotes muscle injury repair. Proc Natl Acad Sci U S A 106, 17475-17480. 10.1073/pnas.0908641106.
[4] Rudnicki, M.A., and Williams, B.O. (2015). Wnt signaling in bone and muscle. Bone 80, 60-66. 10.1016/j.bone.2015.02.009.
[5] Ho, A.T.V., Palla, A.R., Blake, M.R., Yucel, N.D., Wang, Y.X., Magnusson, K.E.G., Holbrook, C.A., Kraft, P.E., Delp, S.L., and Blau, H.M. (2017). Prostaglandin E2 is essential for efficacious skeletal muscle stem-cell function, augmenting regeneration and strength. Proc Natl Acad Sci U S A 114, 6675-6684. 10.1073/pnas.1705420114.
[6] Vinel, C., Lukjanenko, L., Batut, A., Deleruyelle, S., Pradère, J.-P., Le Gonidec, S., Dortignac, A., Geoffre, N., Pereira, O., Karaz, S., et al. (2018). The exerkine apelin reverses age-associated sarcopenia. Nat Med 24, 1360-1371. 10.1038/s41591-018-0131-6.
[7] Palla, A.R., Ravichandran, M., Wang, Y.X., Alexandrova, L., Yang, A.V., Kraft, P., Holbrook, C.A., Schürch, C.M., Ho, A.T.V., and Blau, H.M. (2021). Inhibition of prostaglandin-degrading enzyme 15-PGDH rejuvenates aged muscle mass and strength. Science 371, eabc8059. 10.1126/science.abc8059.
[8] Poli, V. (1998). The role of C/EBP isoforms in the control of inflammatory and native immunity functions. J Biol Chem 273, 29279-29282. 10.1074/jbc.273.45.29279.
[9] Kim, J., Song, H., Heo, H.-R., Kim, J.W., Kim, H.-R., Hong, Y., Yang, S.-R., Han, S.-S., Lee, S.-J., Kim, W.J., et al. (2017). Cadmium-induced ER stress and inflammation are mediated through C/EBP-DDIT3 signaling in human bronchial epithelial cells. Exp Mol Med 49, e372-e372. 10.1038/emm.2017.125.
[10] Varone, E., Pozzer, D., Di Modica, S., Chernorudskiy, A., Nogara, L., Baraldo, M., Cinquanta, M., Fumagalli, S., Villar-Quiles, R.N., De Simoni, M.-G., et al. (2019). SELENON (SEPN1) protects skeletal muscle from saturated fatty acid-induced ER stress and insulin resistance. Redox Biol 24, 101176. 10.1016/j.redox.2019.101176.
[11] Sorgdrager FJH, Vermeiren Y, Van Faassen M, van der Ley C, Nollen EAA, Kema IP, De Deyn PP. (2019). Age- and disease-specific changes of the kynurenine pathway in Parkinson's and Alzheimer's disease. J Neurochem., 151(5):656-668. doi: 10.1111/jnc.14843
[12] Al Saedi, A., Chow, S., Vogrin, S., Guillemin, G.J., and Duque, G. (2022). Association Between Tryptophan Metabolites, Physical Performance, and Frailty in Older Persons. Int J Tryptophan Res 15, 11786469211069951. 10.1177/11786469211069951.
[13] de Bie, J., Guest, J., Guillemin, G.J., and Grant, R. (2016). Central kynurenine pathway shift with age in women. J Neurochem 136, 995-1003. 10.1111/jnc.13496.
[14] Minhas PS, Liu L, Moon PK, Joshi AU, Dove C, Mhatre S, Contrepois K, Wang Q, Lee BA, Coronado M, Bernstein D, Snyder MP, Migaud M, Majeti R, Mochly-Rosen D, Rabinowitz JD, Andreasson KI. (2019). Macrophage de novo NAD+ synthesis specifies immune function in aging and inflammation. Nat Immunol. ;20(1):50-63
[15] Chung, T., Bopp, T., Ward, C., Notarangelo, F.M., Schwarcz, R., Westbrook, R., Xue, Q.-L., Walston, J., and Hoke, A. (2023). Deletion of quinolinate phosphoribosyltransferase gene accelerates frailty phenotypes and neuromuscular decline with aging in a sex-specific pattern. Aging Cell 22, e13849. 10.1111/acel.13849.
[16] S. C. Gandevia. (2001). Spinal and Supraspinal Factors in Human Muscle Fatigue. The American Physiological Society. 10.1152/physrev.2001.81.4.1725.
[17] Orthopedics Made Ridiculously Simple (Medmaster Ridiculously Simple) by M.D. Tetreault Patrice, M.D. Ouellette Hugue, Patrice Tétreault | 9780940780866 | Get Textbooks | New Textbooks | Used Textbooks | College Textbooks - GetTextbooks.com https://www.gettextbooks.com/isbn/9780940780866/.
[18] Compston, A. (2010). Aids to the Investigation of Peripheral Nerve Injuries. Medical Research Council: Nerve Injuries Research Committee. His Majesty's Stationery Office: 1942; pp. 48 (iii) and 74 figures and 7 diagrams; with Aids to the Examination of the Peripheral Nervous System. By Michael O'Brien for the Guarantors of Brain. Saunders Elsevier: 2010; pp. [8] 64 and 94 Figures. Brain 133, 2838-2844. 10.1093/brain/awq270.

### SEQUENCE LISTING

**SEQ ID N°1:** ERO1 primer sequences (hs-ERO1 Forward) : GGCTTCTGGTCAAGGGACAA
**SEQ ID N°2:** ERO1 primer sequences (hs-ERO1 Reverse) : TGCTTGCATGTAGGCCAGATA
**SEQ ID N°3:** plasmid encoding the fusion protein ATF4-mscarlet :

ATATAGCATAAATCAATATTGGCTATTGGCCATTGCATACGTTGTATCCATATCATAATATGTACATT
TATATTGGCTCATGTCCAACATTACCGCCATGTTGACATTGATTATTGACTAGTTATTAATAGTAATC
AATTACGGGGTCATTAGTTCATAGCCCATATATGGAGTTCCGCGTTACATAACTTACGGTAAATGGC
CCGCCTGGCTGACCGCCCAACGACCCCCGCCCATTGACGTCAATAATGACGTATGTTCCCATAGTAA
CGCCAATAGGGACTTTCCATTGACGTCAATGGGTGGAGTATTTACGGTAAACTGCCCACTTGGCAGT
ACATCAAGTGTATCATATGCCAAGTACGCCCCCTATTGACGTCAATGACGGTAAATGGCCCGCCTGG
CATTATGCCCAGTACATGACCTTATGGGACTTTCCTACTTGGCAGTACATCTACGTATTAGTCATCG
CTATTACCATGGTGATGCGGTTTTGGCAGTACATCAATGGGCGTGGATAGCGGTTTGACTCACGGG
GATTTCCAAGTCTCCACCCCATTGACGTCAATGGGAGTTTGTTTTGGCACCAAAATCAACGGGACTT
TCCAAAATGTCGTAACAACTCCGCCCCATTGACGCAAATGGGCGGTAGGCGTGTACGGTGGGAGGT
CTATATAAGCAGAGCTCGTTTAGTGAACCGTCAGATCGCCTGGAGACGCCATCCACGCTGTTTTGAC
CTCCATAGAAGACACCGGGACCGATCCAGCCTCCGCGGCCCCAAGCTTGAATTCTCTACTTTGCCCG
CCCACAGATGTAGTTTTCTCTGCGCGTGTGCGTTTTCCCTCCTCCCCGCCCTCAGGGTCCACGGCCA
CCATGGCGTATTAGGGGCAGCAGTGCCTGCGGCAGCATTGGCCTTTGCAGCGGCGGCAGCAGCACC
AGGCTCTGCAGCGGCAACCCCCAGCGGCTTAAGCCATGGCGCTTCTCACGGCATTCAGCAGCAGCG
TTGCTGTAACCGACAAAGACACCTTCGAATTAAGCACATTCCTCGATTCCAGCAAAGCACCGCAACA
TGACCGAAATGAGCTTCCTGAGCAGCGAGGTGTTGGTGGGGGACTTGATGTCCCCCTTCGACCAGT
CGGGTTTGGGGGCTGAATACCCCTACGACGTGCCCGACTACGCCGGTGGATCAGGGGGAGGATCCG
TCTCCAAAGGGGAAGCGGTCATTAAAGAGTTCATGAGGTTCAAAGTTCATATGGAGGGCAGTATGA
ACGGCCATGAATTTGAAATCGAGGGCGAGGGGGAGGGACGCCCTTACGAAGGGACACAAACAGCCA
AACTGAAGGTTACGAAAGGTGGGCCACTTCCCTTTAGTTGGGATATACTGTCACCTCAATTTATGTA
CGGCAGCCGAGCCTTCATTAAACATCCCGCTGACATTCCGGATTACTATAAGCAGTCCTTTCCAGAG
GGGTTTAAATGGGAGCGGGTTATGAATTTCGAGGATGGCGGGGCCGTAACTGTCACGCAGGATACC
AGTCTGGAAGATGGTACCCTGATTTATAAGGTTAAGCTCAGAGGAACGAACTTCCCGCCAGATGGTC
CCGTAATGCAAAAAAAGACGATGGGTTGGGAGGCATCAACTGAAAGATTGTATCCTGAGGATGGTG
TCCTTAAGGGTGATATTAAAATGGCTCTGAGGCTGAAGGATGGCGGAAGGTATTTGGCAGATTTCA
AAACTACATATAAGGCCAAGAAGCCTGTTCAGATGCCAGGTGCCTATAATGTGGACAGAAAACTTGA
CATTACATCACACAACGAGGATTATACCGTCGTGGAGCAATATGAGAGAAGTGAGGGCCGACATAGT

ACCGGCGGCATGGATGAACTCTATAAGGTCGACGGTGGATCAGGGGGACCCGCCGCCAAGCGCGTG
AAGCTGGACTAATCTAGAATCGATAAAATAAAAGATTTTATTTAGTCTCCAGAAAAAGGGGGGAATG
AAAGACCCCACCTGTAGGTTTGGCAAGCTAGCTTAAGTAACGCCATTTTGCAAGGCATGGAAAAATA
CATAACTGAGAATAGAGAAGTTCAGATCAAGGTCAGGAACAGATGGAACAGCTGAATATGGGCCAA
ACAGGATATCTGTGGTAAGCAGTTCCTGCCCCGGCTCAGGGCCAAGAACAGATGGAACAGCTGAAT
ATGGGCCAAACAGGATATCTGTGGTAAGCAGTTCCTGCCCCGGCTCAGGGCCAAGAACAGATGGTC
CCCAGATGCGGTCCAGCCCTCAGCAGTTTCTAGAGAACCATCAGATGTTTCCAGGGTGCCCCAAGGA
CCTGAAATGACCCTGTGCCTTATTTGAACTAACCAATCAGTTCGCTTCTCGCTTCTGTTCGCGCGCT
TCTGCTCCCCGAGCTCAATAAAAGAGCCCACAACCCCTCACTCGGGGCGCCAGTCCTCCGATTGACT
GAGTCGCCCGGGTACCCGTGTATCCAATAAACCCTCTTGCAGTTGCATCCGACTTGTGGTCTCGCTG
TTCCTTGGGAGGGTCTCCTCTGAGTGATTGACTACCCGTCAGCGGGGGTCTTTCATTTGGGGGCTC
GTCCGGGATCGGGAGACCCCTGCCCAGGGACCACCGACCCACCACCGGGAGGTAAGCTGGCTGCCT
CGCGCGTTTCGGTGATGACGGTGAAAACCTCTGACACATGCAGCTCCCGGAGACGGTCACAGCTTG
TCTGTAAGCGGATGCCGGGAGCAGACAAGCCCGTCAGGGCGCGTCAGCGGGTGTTGGCGGGTGTC
GGGGCGCAGCCATGACCCAGTCACGTAGCGATAGCGGAGTGTATACTGGCTTAACTATGCGGCATC
AGAGCAGATTGTACTGAGAGTGCACCATATGCGGTGTGAAATACCGCACAGATGCGTAAGGAGAAA
ATACCGCATCAGGCGCTCTTCCGCTTCCTCGCTCACTGACTCGCTGCGCTCGGTCGTTCGGCTGCGG
CGAGCGGTATCAGCTCACTCAAAGGCGGTAATACGGTTATCCACAGAATCAGGGGATAACGCAGGA
AAGAACATGTGAGCAAAAGGCCAGCAAAAGGCCAGGAACCGTAAAAAGGCCGCGTTGCTGGCGTTT
TTCCATAGGCTCCGCCCCCCTGACGAGCATCACAAAAATCGACGCTCAAGTCAGAGGTGGCGAAACC
CGACAGGACTATAAAGATACCAGGCGTTTCCCCCTGGAAGCTCCCTCGTGCGCTCTCCTGTTCCGAC
CCTGCCGCTTACCGGATACCTGTCCGCCTTTCTCCCTTCGGGAAGCGTGGCGCTTTCTCATAGCTCA
CGCTGTAGGTATCTCAGTTCGGTGTAGGTCGTTCGCTCCAAGCTGGGCTGTGTGCACGAACCCCCC
GTTCAGCCCGACCGCTGCGCCTTATCCGGTAACTATCGTCTTGAGTCCAACCCGGTAAGACACGACT
TATCGCCACTGGCAGCAGCCACTGGTAACAGGATTAGCAGAGCGAGGTATGTAGGCGGTGCTACAG
AGTTCTTGAAGTGGTGGCCTAACTACGGCTACACTAGAAGGACAGTATTTGGTATCTGCGCTCTGCT
GAAGCCAGTTACCTTCGGAAAAAGAGTTGGTAGCTCTTGATCCGGCAAACAAACCACCGCTGGTAGC
GGTGGTTTTTTTGTTTGCAAGCAGCAGATTACGCGCAGAAAAAAAGGATCTCAAGAAGATCCTTTGA
TCTTTTCTACGGGGTCTGACGCTCAGTGGAACGAAAACTCACGTTAAGGGATTTTGGTCATGAGATT
ATCAAAAAGGATCTTCACCTAGATCCTTTTAAATTAAAAATGAAGTTTTAAATCAATCTAAAGTATAT
ATGAGTAAACTTGGTCTGACAGTTACCAATGCTTAATCAGTGAGGCACCTATCTCAGCGATCTGTCT
ATTTCGTTCATCCATAGTTGCCTGACTCCCCGTCGTGTAGATAACTACGATACGGGAGGGCTTACCA
TCTGGCCCCAGTGCTGCAATGATACCGCGAGACCCACGCTCACCGGCTCCAGATTTATCAGCAATAA
ACCAGCCAGCCGGAAGGGCCGAGCGCAGAAGTGGTCCTGCAACTTTATCCGCCTCCATCCAGTCTAT
TAATTGTTGCCGGGAAGCTAGAGTAAGTAGTTCGCCAGTTAATAGTTTGCGCAACGTTGTTGCCATT

GCTGCAGGCATCGTGGTGTCACGCTCGTCGTTTGGTATGGCTTCATTCAGCTCCGGTTCCCAACGAT
CAAGGCGAGTTACATGATCCCCCATGTTGTGCAAAAAAGCGGTTAGCTCCTTCGGTCCTCCGATCGT
TGTCAGAAGTAAGTTGGCCGCAGTGTTATCACTCATGGTTATGGCAGCACTGCATAATTCTCTTACT
GTCATGCCATCCGTAAGATGCTTTTCTGTGACTGGTGAGTACTCAACCAAGTCATTCTGAGAATAGT
GTATGCGGCGACCGAGTTGCTCTTGCCCGGCGTCAACACGGGATAATACCGCGCCACATAGCAGAA
CTTTAAAAGTGCTCATCATTGGAAAACGTTCTTCGGGGCGAAAACTCTCAAGGATCTTACCGCTGTT
GAGATCCAGTTCGATGTAACCCACTCGTGCACCCAACTGATCTTCAGCATCTTTTACTTTCACCAGC
GTTTCTGGGTGAGCAAAAACAGGAAGGCAAAATGCCGCAAAAAAGGGAATAAGGGCGACACGGAAA
TGTTGAATACTCATACTCTTCCTTTTTCAATATTATTGAAGCATTTATCAGGGTTATTGTCTCATGAG
CGGATACATATTTGAATGTATTTAGAAAAATAAACAAATAGGGGTTCCGCGCACATTTCCCCGAAAA
GTGCCACCTGACGTCTAAGAAACCATTATTATCATGACATTAACCTATAAAAATAGGCGTATCACGA
GGCCCTTTCGTCTTCAAGAATTCATACCAGATCACCGAAAACTGTCCTCCAAATGTGTCCCCCTCACA
CTCCCAAATTCGCGGGCTTCTGCCTCTTAGACCACTCTACCCTATTCCCCACACTCACCGGAGCCAAA
GCCGCGGCCCTTCCGTTTCTTTGCTTTTGAAAGACCCCACCCGTAGGTGGCAAGCTAGCTTAAGTAA
CGCCACTTTGCAAGGCATGGAAAAATACATAACTGAGAATAGAAAAGTTCAGATCAAGGTCAGGAAC
AAAGAAACAGCTGAATACCAAACAGGATATCTGTGGTAAGCGGTTCCTGCCCCGGCTCAGGGCCAA
GAACAGATGAGACAGCTGAGTGATGGGCCAAACAGGATATCTGTGGTAAGCAGTTCCTGCCCCGGC
TCGGGGCCAAGAACAGATGGTCCCCAGATGCGGTCCAGCCCTCAGCAGTTTCTAGTGAATCATCAGA
TGTTTCCAGGGTGCCCCAAGGACCTGAAAATGACCCTGTACCTTATTTGAACTAACCAATCAGTTCG
CTTCTCGCTTCTGTTCGCGCGCTTCCGCTCTCCGAGCTCAATAAAAGAGCCCACAACCCCTCACTCG
GCGCGCCAGTCTTCCGATAGACTGCGTCGCCCGGGTACCCGTATTCCCAATAAAGCCTCTTGCTGTT
TGCATCCGAATCGTGGTCTCGCTGTTCCTTGGGAGGGTCTCCTCTGAGTGATTGACTACCCACGACG
GGGGTCTTTCATTTGGGGGCTCGTCCGGGATTTGGAGACCCCTGCCCAGGGACCACCGACCCACCA
CCGGGAGGTAAGCTGGCCAGCAACTTATCTGTGTCTGTCCGATTGTCTAGTGTCTATGTTTGATGTT
ATGCGCCTGCGTCTGTACTAGTTAGCTAACTAGCTCTGTATCTGGCGGACCCGTGGTGGAACTGAC
GAGTTCTGAACACCCGGCCGCAACCCTGGGAGACGTCCCAGGGACTTTGGGGGCCGTTTTTGTGGC
CCGACCTGAGGAAGGGAGTCGATGTGGAATCCGACCCCGTCAGGATATGTGGTTCTGGTAGGAGAC
GAGAACCTAAAACAGTTCCCGCCTCCGTCTGAATTTTTGCTTTCGGTTTGGAACCGAAGCCGCGCGT
CTTGTCTGCTGCAGCGCTGCAGCATCGTTCTGTGTTGTCTCTGTCTGACTGTGTTTCTGTATTTGTC
TGAAAATTAGGGCCAGACTGTTACCACTCCCTTAAGTTTGACCTTAGGTCACTGGAAAGATGTCGAG
CGGATCGCTCACAACCAGTCGGTAGATGTCAAGAAGAGACGTTGGGTTACCTTCTGCTCTGCAGAAT
GGCCAACCTTTAACGTCGGATGGCCGCGAGACGGCACCTTTAACCGAGACCTCATCACCCAGGTTAA
GATCAAGGTCTTTTCACCTGGCCCGCATGGACACCCAGACCAGGTCCCCTACATCGTGACCTGGGAA
GCCTTGGCTTTTGACCCCCCTCCCTGGGTCAAGCCCTTTGTACACCCTAAGCCTCCGCCTCCTCTTC
CTCCATCCGCCCCGTCTCTCCCCCTTGAACCTCCTCGTTCGACCCCGCCTCGATCCTCCCTTTATCCA

GCCCTCACTCCTTCTCTAGGCGCCGGAATTCCGATCTGATAGCTTGCCACAACCCGTACCAAAGATG
GATAGATCCGGAAAGCCTGAACTCACCGCGACGTCTGTCGAGAAGTTTCTGATCGAAAAGTTCGACA
GCGTCTCCGACCTGATGCAGCTCTCGGAGGGCGAAGAATCTCGTGCTTTCAGCTTCGATGTAGGAG
GGCGTGGATATGTCCTGCGGGTAAATAGCTGCGCCGATGGTTTCTACAAAGATCGTTATGTTTATCG
GCACTTTGCATCGGCCGCGCTCCCGATTCCGGAAGTGCTTGACATTGGGGAATTCAGCGAGAGCCT
GACCTATTGCATCTCCCGCCGTGCACAGGGTGTCACGTTGCAAGACCTGCCTGAAACCGAACTGCCC
GCTGTTCTGCAGCCGGTCGCGGAGGCCATGGATGCGATCGCTGCGGCCGATCTTAGCCAGACGAGC
GGGTTCGGCCCATTCGGACCGCAAGGAATCGGTCAATACACTACATGGCGTGATTTCATATGCGCG
ATTGCTGATCCCCATGTGTATCACTGGCAAACTGTGATGGACGACACCGTCAGTGCGTCCGTCGCGC
AGGCTCTCGATGAGCTGATGCTTTGGGCCGAGGACTGCCCCGAAGTCCGGCACCTCGTGCACGCGG
ATTTCGGCTCCAACAATGTCCTGACGGACAATGGCCGCATAACAGCGGTCATTGACTGGAGCGAGG
CGATGTTCGGGGATTCCCAATACGAGGTCGCCAACATCTTCTTCTGGAGGCCGTGGTTGGCTTGTA
TGGAGCAGCAGACGCGCTACTTCGAGCGGAGGCATCCGGAGCTTGCAGGATCGCCGCGGCTCCGGG
CGTATATGCTCCGCATTGGTCTTGACCAACTCTATCAGAGCTTGGTTGACGGCAATTTCGATGATGC
AGCTTGGGCGCAGGGTCGATGCGACGCAATCGTCCGATCCGGAGCCGGGACTGTCGGGCGTACACA
AATCGCCCGCAGAAGCGCGGCCGTCTGGACCGATGGCTGTGTAGAAGTACTCGCCGATAGTGGAAA
CCGACGCCCCAGCACTCGTCCGAGGGCAAAGGAATAGAGTAGATGCCGACCGAACAAGAGCTGATT
TCGAGAACGCCTCAGCCAGCAACTCGCGCGAGCCTAGCAAGGCAAATGCGAGAGAACGGCCTTACG
CTTGGTGGCACAGTTCTCGTCCACAGTTCGCTAAGCTCGCTCGGCTGGGTCGCGGGAGGGCCGGTC
GCAGTGATTCAGGCCCTTCTGGATTGTGTTGGTCCCCAGGGCACGATTGTCATGCCCACGCACTCG
GGTGATCTGACTGATCCCGCAGATTGGAGATCGCCGCCCGTGCCTGCCGATTGGGTGCAGATCTCT
AGAGCTCGCTGATCCGGCCATTAGCCATATTATTCATTGGTT

## Claims

1. An *in vitro* method for prognosticating and/or diagnosing a muscle dysfunction in a subject, comprising:
a) measuring the amounts of kynurenine, tryptophan, kynurenic acid and quinolinic acid in a biological sample from the subject, and determining the ratios (i) Kynurenine:Tryptophan and/or Kynurenic acid:Tryptophan, and (ii) Quinolinic acid:Tryptophan;
b) comparing the ratios determined in step a) to a reference value;
c) attributing (i) an increase of the ratio Kynurenine:Tryptophan and/or Kynurenic acid:Tryptophan from the reference value, and (ii) no deviation of the ratio of Quinolinic acid:Tryptophan from the reference value to a muscle dysfunction in the subject.

2. An *in vitro* method for prognosticating and/or diagnosing a muscle dysfunction in a subject, comprising:
a) measuring the amounts of kynurenine, tryptophan, kynurenic acid and quinolinic acid in a biological sample from the subject, and determining the ratios (i) Kynurenine:Quinolinic acid and/or Kynurenic acid:Quinolinic acid, and (ii) Quinolinic acid:Tryptophan;
b) comparing the ratios determined in step a) to a reference value;
c) attributing (i) an increase of the ratio Kynurenine:Quinolinic acid and/or Kynurenic acid:Quinolinic acid from the reference value, and (ii) no deviation of the ratio of Quinolinic acid:Tryptophan from the reference value to a muscle dysfunction in the subject.

3. An *in vitro* method for evaluating the muscle efficiency in a subject, said method comprising:
a) measuring the amounts of kynurenine, tryptophan, kynurenic acid and quinolinic acid in a biological sample from the subject, and determining the ratios (i) Kynurenine:Tryptophan and/or Kynurenic acid:Tryptophan, and (ii) Quinolinic acid:Tryptophan;
b) comparing the ratios determined in step a) to a reference value;
c) attributing (i) an increase of the ratio Kynurenine:Tryptophan and/or Kynurenic acid:Tryptophan from the reference value, and (ii) no deviation of the ratio of Quinolinic acid:Tryptophan from the reference value to a weak muscle efficiency in the subject.

4. An *in vitro* method for evaluating the muscle efficiency in a subject, said method comprising:
a) measuring the amounts of kynurenine, tryptophan, kynurenic acid and quinolinic acid in a biological sample from the subject, and determining the ratios (i) Kynurenine:Quinolinic acid and/or Kynurenic acid:Quinolinic acid, and (ii) Quinolinic acid:Tryptophan;
b) comparing the ratios determined in step a) to a reference value;
c) attributing (i) an increase of the ratio Kynurenine:Quinolinic acid and/or Kynurenic acid:Quinolinic acid from the reference value, and (ii) no deviation of the ratio of Quinolinic acid:Tryptophan from the reference value to a weak muscle efficiency in the subject.

5. The method according to claim 1, comprising:
a') in step a), also measuring the amounts of oxidized nicotinamide adenine dinucleotide (NAD+), total nicotinamide adenine dinucleotide (NAD) and adenosine triphosphate (ATP), and determining the ratio of NAD+: total NAD;
b') in step b), also comparing the ratio of NAD+: total NAD and the amount of ATP obtained in step a) to a reference value;
c') in step c), attributing (i) an increase of the ratio Kynurenine:Tryptophan and/or Kynurenic acid:Tryptophan from the reference value, (ii) no deviation of the ratio of Quinolinic acid:Tryptophan from the reference value, and (iii) a decrease of the ratio of NAD+: total NAD and/or of the amount of ATP from the reference value, to a muscle dysfunction in the subject.

6. The method according to claim 2 comprising:
a') in step a), also measuring the amounts of oxidized nicotinamide adenine dinucleotide (NAD+), total nicotinamide adenine dinucleotide (NAD) and adenosine triphosphate (ATP), and determining the ratio of NAD+: total NAD;
b') in step b), also comparing the ratio of NAD+: total NAD and the amount of ATP obtained in step a) to a reference value;
c') in step c), attributing (i) an increase of the ratios Kynurenine:Quinolinic acid and/or Kynurenic acid:Quinolinic acid from the reference value, (ii) no deviation of the ratio of Quinolinic acid:Tryptophan from the reference value, and (iii) a decrease of the ratio of NAD+: total NAD and/or of the amount of ATP from the reference value, to a muscle dysfunction in the subject.

7. The method according to any of claims 1, 2, 5 and 6, wherein the muscle dysfunction results from:
i) a disease chosen from the group consisting of congenital myopathies, sarcopenia, cachexia, disuse atrophy, mitochondrial myopathies, metabolic myopathies, glycogen storage diseases, muscular dystrophies such as facioscapulohumeral muscular dystrophy (FSHD), myasthenia gravis, congenital myasthenic syndromes, inflammatory myopathies such as polymyositis, inclusion body myositis, myotonic dystrophy, cachexia, chronic fatigue syndrome, fibromyalgia, Guillain-Barré syndrome, spinal muscular atrophy, amyotrophic lateral sclerosis (ALS), peripheral neuropathy, drug-related myopathies, virus-related myopathies such as COVID-19 related, toxin-related flaccid paralysis such as Botulinum toxins related, and/or
ii) muscle with limited mobility, muscle trauma, muscle fatigue, muscle weakness, suboptimal muscle performance.

8. The method according to any of claims 1, 2 and 5 to 7, wherein the muscle dysfunction results from a congenital myopathy chosen among SELENON-related myopathy, RYR1-related myopathy, and desminopathy.

9. The method according to any of claims 3 and 4, wherein the weak muscle efficiency results from a muscle disease, muscle trauma, muscle weakness, suboptimal muscle performance, muscle with limited mobility and/or muscle fatigue.

10. The method according to any of the preceding claims, wherein the biological sample is blood, urine, saliva or muscle sample.

11. The method according to any of claims 1 and 5 to 8, wherein the ratio Kynurenine:Tryptophan and/or Kynurenic acid:Tryptophan in the sample from the subject is increased by at least 10%, preferably by at least 15%, preferably by at least 20%, more preferably by at least 25%, even more preferably by at least 30% compared to the reference value.

12. The method according to any of claims 2 and 5 to 8 wherein the ratio Kynurenine:Quinolinic acid and/or Kynurenic acid:Quinolinic acid in the sample from the subject is increased by at least 10%, preferably by at least 15%, preferably by at least 20%, more preferably by at least 25%, even more preferably by at least 30% compared to the reference value.

13. The method according to any of claims 5 to 8 and 10 to 12, wherein the ratio of NAD+: total NAD and/or the amount of ATP in the sample from the subject is decreased by at least 10%, preferably by at least 15%, preferably by at least 20%, more preferably by at least 25%, even more preferably by at least 30% compared to the reference value.

14. The method according to any of claims 1, 2, 5 to 8 and 10 to 13, wherein the reference value represents a healthy subject.

15. The method according to any of claims 1, 2, 5 to 8 and 10 to 14, further comprises a step of subjecting the subject having a muscle dysfunction to a medical treatment, such as a therapeutic treatment or a prophylactic treatment or physical intervention.

16. An *in vitro* method for monitoring a change in the diagnosis and/or prognosis of a muscle dysfunction in a subject, comprising:
a) implementing the method according to any of claims 1, 2, 5 to 8, and 10 to 15, to the subject at one or more successive time points, whereby the diagnosis and/or prognosis of muscle dysfunction in the subject is determined at said successive time points; and
b) finding a presence or absence of a change between the diagnosis and/or prognosis of a muscle dysfunction in the subject at said successive time points as determined in step a).

17. An *in vitro* method for monitoring a change in the muscle efficiency in a subject, comprising:
a) implementing the method of any of claims 3 or 4 to the subject at one or more successive time points, whereby the muscle efficiency in the subject is determined at said successive time points; and
b) finding a presence or absence of a change between the muscle efficiency in the subject at said successive time points as determined in step a).

18. A kit for prognosticating and/or diagnosing a muscle dysfunction and/or for evaluating muscle efficiency in a subject, comprising:
- one or more reagents for measuring the amounts of markers in a biological sample, wherein said markers are kynurenine, tryptophan, kynurenic acid, quinolinic acid, NAD+, NADH and/or ATP
- one or more containers suitable for mixing the biological sample with the one or more reagents,
- optionally, one or more surfaces which have an affinity for the markers which may be brought into contact with the biological sample,
- optionally, one or more suitable washing solutions,
- optionally, one or more components necessary for the detection of the markers, for example an enzyme and/or a substrate.
